(19) Europäisches Patentamt
European Patent Office
Office européen des brevets

(11) **EP 4 553 070 A1**

(12) **EUROPEAN PATENT APPLICATION**

(43) Date of publication:
**14.05.2025 Bulletin 2025/20**

(21) Application number: **23208210.7**

(22) Date of filing: **07.11.2023**

(51) International Patent Classification (IPC):
***C07D 307/93*** (2006.01)    ***A01N 65/12*** (2009.01)
***A61K 31/343*** (2006.01)

(52) Cooperative Patent Classification (CPC):
**C07D 307/93; A61K 31/343**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Coöperatie Koninklijke Cosun U.A.
4814 NE Breda (NL)**

(72) Inventors:
• **VAN DEN BERGH, Johan
4814 NE Breda (NL)**
• **HUIJGEN, Wouter Johannes Joseph
4814 NE Breda (NL)**

(74) Representative: **Nederlandsch Octrooibureau
P.O. Box 29720
2502 LS The Hague (NL)**

(54) **PROCESS FOR THE RECOVERY OF SESQUITERPENE LACTONES**

(57)    The present invention relates to a process for recovering sesquiterpene lactones using non-ionic polymer resin adsorption.

Processed by Luminess, 75001 PARIS (FR)

## Description

### Field of the invention

The present invention relates to a process for recovering sesquiterpene lactones from an aqueous stream comprising sesquiterpene lactones, such as a chicory root extract or a sesquiterpene lactone-containing derivative thereof. The invention further relates to a composition comprising sesquiterpene lactones obtainable from the process and to an inulin composition which comprises low concentrations of impurities such as sesquiterpene lactones.

### Background of the invention

Chicory plants (such as *Cichorium intybus* L.) are known for their pest resistance. Certain compounds found in the leaves and the roots of the chicory plant are known to act as feeding deterrents for insects. Moreover, these compounds defend the plant against herbivory. Among the compounds found in the leaves and the roots of the chicory plant are the so-called sesquiterpene lactones or 'bitter components'. Sesquiterpene lactones may include but are not limited to guaianolides, eudesmanolides, or germacranolides. The guaianolides may include at least one of lactucin, 8-deoxylactucin, and lactucopicrin. Recently, it has been found that chicory plant extracts are effective against *Stemphylium beticola* and/or *Cercospora beticola* on crops, such as sugar beet and potato. This effect is believed to be due to sesquiterpene lactones contained in the extract.

At present, the chicory crop is mainly used for the extraction of inulin. Inulins are linear chains consisting of fructose units connected via a $\beta$ (2-1) bond, which typically have a terminal glucose unit. The number of monosaccharide units is commonly referred to as the degree of polymerization (DP) and typically ranges from 3 to 80. Chicory inulin typically comprises 20-35% DP3-DP9 inulin and 65-80% DP10 and higher inulin. Inulin is used in the food industry because of its attractive nutritional, binding, gelling and physiological properties while chemically modified inulin derivatives find applications in the non-food industry, such as in personal care products.

Industrial scale inulin production typically involves hot water extraction of sliced plant material, followed by purification of the crude extract and isolation of the inulin. The production of inulin from chicory plants, in particular from chicory root from *Cichorium intybus* L., poses specific challenges because of the presence of large amounts of sesquiterpene lactones, which are not significantly present in other inulin sources such as e.g. Jerusalem Artichoke. The sesquiterpene lactones, and more specifically the lactucin-type sesquiterpene lactones, have an undesirable, bitter taste. In the past, these lactones were regarded as being of little commercial value and thus present inulin production processes were designed to remove sesquiterpene lactones that result from the extraction process without any regard to their potential use.

Purification of the crude chicory root extract is commonly done by ion-exchange treatment combined with active carbon filtration. The present inventors have found that while sesquiterpene lactones are effectively removed from the chicory extract by ion-exchange resin, they are also degraded under the conditions necessary to regenerate the ion-exchange resin, which employs strong acids or bases. Thus, the sesquiterpene lactones cannot be recovered from such ion-exchange resins. Similarly, the residual sesquiterpene lactones passing to the active carbon treatment stage will be adsorbed on the active carbon resin, but the present inventors have found that desorption from the active carbon resin is particularly problematic. Furthermore the active carbon is conventionally regenerated by thermal treatment which destroys the sesquiterpene lactones.

As a consequence, currently sesquiterpene lactones can only be isolated from chicory in reasonable yields using dedicated extraction processes, which are not integrated with other chicory valorisation processes such as inulin production and are not cost-effective to operate at a large scale. It would be desirable if sesquiterpene lactones could be obtained from chicory plants in an industrially viable process which allows the sesquiterpene lactones to be used for various purposes, for example as a fungicide or an insecticide. Ideally, such sesquiterpene lactone production could be integrated with conventional inulin production.

Finally, known sesquiterpene lactone compositions produced by extraction typically comprise high amounts of sugars, which can be undesirable for certain applications, for example in crop protection as the high sugar content may attract certain pathogens like fungi and molds.

It is an object of the present invention to provide a process for recovering sesquiterpene lactones from an aqueous liquid comprising sesquiterpene lactones and one or more of sugars, minerals, inulin and polyphenols, such as a chicory extract.

It is an object of the present invention to provide a process for recovering sesquiterpene lactones from an aqueous liquid comprising sesquiterpene lactones and one or more of sugars, minerals, inulin and polyphenols, such as a chicory extract, which can be operated at an industrial scale in an economically viable manner and/or which is integrated with inulin production.

It is an object of the present invention to provide sesquiterpene lactone compositions having improved purity, such as a reduced sugar contents.

It is an object of the present invention to provide inulin compositions having improved purity, such as a reduced sesquiterpene lactone and/or polyphenol content.

**Summary of the invention**

The inventors have found that one or more of these objects may be achieved by using a non-ionic polymer resin. As is shown in the appended examples, it was found that sesquiterpene lactones contained in complex mixtures such as chicory extracts may be highly efficiently and selectively adsorbed on and desorbed from non-ionic polymer resins. Furthermore, it was found that non-ionic polymer resins even allow for separating polyphenols from sesquiterpene lactones, either due to a high selectivity in adsorption and/or because polyphenols adsorbed on such resins may be selectively desorbed from the resin while leaving sesquiterpene lactones on the resin, such that extremely low polyphenol sesquiterpene lactone compositions can be obtained and if desired polyphenols could even be separately recovered. The present method allows for facile integration in an existing inulin production process.

Thus, in a first aspect the present invention provides a process for recovering sesquiterpene lactones, comprising the steps of:

(a) providing an aqueous liquid comprising sesquiterpene lactones and one or more of sugars, minerals, inulin and polyphenols;

(b) subjecting the aqueous liquid of step (a) to a resin adsorption step using a stationary phase comprising a first non-ionic polymer resin, thereby obtaining a sesquiterpene lactones loaded resin and an adsorption eluate; and

(c) desorbing sesquiterpene lactones from the loaded resin of step (b) and collecting a desorption eluate comprising sesquiterpene lactones.

In preferred embodiments of the invention the aqueous liquid provided in step (a) is a plant material extract or originates from a plant material extract, preferably the aqueous liquid provided in step (a) is a plant material extract from a plant of the Asteraceae family or originates from a plant material extract from a plant of the Asteraceae family, such as chicory.

In a preferred embodiment of the invention, step (b) comprises subjecting at least 10 bed volumes, preferably at least 20 bed volumes, more preferably at least 30 bed volumes of the aqueous liquid provided in step (a) to resin adsorption using a stationary phase which is a packed or expanded bed comprising or consisting of the non-ionic polymer resin.

In preferred embodiments of the invention, the process comprises a washing step between steps (b) and (c), wherein the resin is washed with an aqueous solvent comprising more than 80 vol% water.

Step (c) of the process of the present invention preferably comprises desorbing sesquiterpene lactones with a solvent comprising an organic solvent and optionally water.

In preferred embodiments, the process of the present invention further comprises a step (e) subjecting the adsorption eluate of step (b) to one or more of the following treatments:

- (e1) nanofiltration;

- (e2) precipitation or crystallization;

- (e3) ion-exchange resin treatment and/or active carbon treatment; and

- (e4) electrodialysis.

In embodiments, the method of the present invention further comprises a step (f) of concentrating the adsorption eluate as obtained from step (b) or after the one or more treatments of step (e).

In some embodiments of the invention, the process comprises a step

(g) subjecting a polyphenol-containing stream to a resin adsorption step using a stationary phase comprising a non-ionic polymer resin, thereby obtaining a polyphenol loaded resin and an eluate,

wherein the polyphenol-containing stream is selected from

- the aqueous liquid of step (a); and/or

- the washing liquid of the optional washing step described herein;

- and/or the adsorption eluate of step (b) or a derivative thereof.

In another aspect, the invention provides a sesquiterpene lactone-containing composition obtainable by the process described herein.

In another aspect the invention provides a sesquiterpene lactone composition comprising sesquiterpene lactones and at least one of polyphenols and sugars,

• wherein the ratio (w/w) of sesquiterpene lactones to sugars is at least 2:1, preferably at least 5:1, more preferably at least 10:1; and/or

• wherein the ratio (w/w) of sesquiterpene lactones to polyphenols is at least 10:1, preferably at least 25:1, more preferably at least 100:1, most preferably at least 1000:1.

In another aspect, the invention provides an inulin-rich composition obtainable by the process described herein.

In another aspect the invention provides an inulin-rich composition comprising inulin, preferably chicory inulin, wherein the composition comprises less than 1000 mg sesquiterpene lactones per kg of inulin, preferably less than 500 mg sesquiterpene lactones per kg of inulin, more preferably less than 200 mg sesquiterpene lactones per kg of inulin.

## Description of embodiments

The expression "comprise" and variations thereof, such as, "comprises" and "comprising" as used herein should be construed in an open, inclusive sense, meaning that the embodiment described includes the recited features, but that it does not exclude the presence of other features, as long as they do not render the embodiment unworkable.

The expressions "one embodiment", "a particular embodiment", "an embodiment" etc. as used herein should be construed to mean that a particular feature, structure or characteristic described in connection with the embodiment is included in at least one embodiment. Thus, the appearances of such expressions in various places throughout this specification do not necessarily all refer to the same embodiment. Furthermore, the particular features, structures, or characteristics may be combined in any suitable manner in one or more embodiments. For example, certain features of the disclosure which are described herein in the context of separate embodiments are also explicitly envisaged in combination in a single embodiment.

The singular forms "a," "an," and "the" as used herein should be construed to include plural referents unless the content clearly dictates otherwise. It should also be noted that the term "or" is generally employed in its broadest sense, that is as meaning "and/or" unless the content clearly dictates otherwise.

Whenever reference is made throughout this document to a compound which is a salt, this should be construed to include the anhydrous form as well as any solvates (in particular hydrates) of this compound, unless explicitly defined otherwise.

The term 'chicory', as used herein, refers to plants of the genus *Cichorium,* including plants of the species *Cichorium endivia,* plants of the species *Cichorium intybus,* varieties thereof, and hybrids thereof. The term "chicory" includes plants of the species *Cichorium intybus,* including "wild improved" chicories, "Barbe de Capucin" chicories, "sugar loaf" chicories, "Chioggia" chicories, "cicorino" chicories, "Verona" chicories, "Catalonia" chicories, "Treviso" chicories, "Variegato di Castelfranco" chicories, "Witloof" chicories (e.g., "Brussels" chicory or "chicon" chicory), "Soncino" chicories, "red" chicories (e.g., radicchios), "Industrial" chicories (e.g., chicories intended for roasting and for sugars), "fodder" or "game" chicories, and hybrids thereof. The term "chicory" includes plants of the species *Cichorium endivia,* such as curly endive, also referred to as frisee (var. *crispum*), escarole, or broad-leaved endive (var. *latifolia),* and hybrids thereof. In preferred embodiments of the invention, the chicory is *Cichorium intybus.* In an even more preferred embodiment the chicory is a *Cichorium intybus L.* var. *sativum* (root chicory) extract, such as *Cichorium intybus* L. var. *sativum* DC. Sesquiterpene lactones are typically found in various parts of chicory plants, such as the leaves, roots, flowers and seeds. In a preferred embodiment of the invention the chicory extract referred to herein is obtained from chicory plant parts selected from the group consisting of leaves, roots, flowers, seeds and combinations thereof, more preferably from the leaves or the roots, most preferably from the root. Thus, the chicory extract referred to herein is most preferably a *Cichorium intybus* root extract, such as a *Cichorium intybus L.* var. *sativum* root extract.

As used herein, the term *'inulin'* refers to polymers composed of linear chains of fructose units connected via a $\beta$ (2-1) glycosidic bond which may have a terminal glucose unit, wherein the number of monosaccharide units in an inulin molecule (commonly referred to as the degree of polymerization, DP) is at least 3. Inulins with a terminal glucose (alpha-D-glucopyranosyl-[beta-D-fructofuranosyl](n-1)-D-fructofuranosides) are referred to herein as GpyFn inulins. Inulins without a terminal glucose (beta-D-fructopyranosyl-[D-fructofuranosyl](n-1)-D-fructofuranosides) are referred to herein as FpyFn. The wording 'inulin comprising GpyFn inulins' as used herein refers to inulin having GpyFn inulin chains with different degree of polymerization. Likewise, the wording 'inulin comprising FpyFn inulins' as used herein refers to inulin having FpyFn inulin chains with different degree of polymerization.

As is known to the skilled person, naturally occurring inulin mainly comprises GpyFn, but FpyFn may be present as a product of the hydrolysis of inulins, for example resulting from spontaneous degradation or resulting from a chemical or an enzymatic treatment wherein inulins are hydrolyzed.

A suitable method to determine the inulin content, defined as the combined amount (i.e. the sum) of DP3, DP4, DP5 and >DP5 is by a chromatographic method such as gel permeation chromatography coupled to a refractive index detector (GPC-RI). A preferred method to determine the inulin content is in accordance with the GPC-RI protocol described herein. Thus, in embodiments, the methods and products of the invention are provided, having the inulin content characteristics as described herein when determined in accordance with the GPC-RI protocol described herein.

A suitable method to determine the inulin chain length distribution is by a chromatographic method, such as high performance anion exclusion chromatography coupled to pulsed amperometric detection (HPAEC-PAD). A preferred method to determine the inulin chain length distribution is in accordance with the HPAEC-PAD protocol described herein. Thus, in embodiments, the methods and products of the invention are provided, having the inulin chain length distribution characteristics as described herein when determined in accordance with the HPAEC-PAD protocol described herein.

As used herein, the term 'sesquiterpene lactones' refers to one or more of lactucin, dihydro-lactucin, 8-deoxylactucin-15-oxalate, 8-deoxylactucin, dihydro-8-deoxylactucin, lactucopicrin-15-oxalate, dihydro-lactucopicrin-15-oxalate, dihydro-lactucopicrin, lactucopicrin, dihydro-lactucin-15-oxalate, lactucin-15-oxalate and dihydro-8-deoxy-lactucin-15-oxalate. The sesquiterpene lactone content as used herein concerns the combined amount (i.e. the sum) of lactucin, dihydro-lactucin, 8-deoxylactucin-15-oxalate, 8-deoxylactucin, dihydro-8-deoxylactucin, lactucopicrin-15-oxalate, dihydro-lactucopicrin-oxalate, dihydro-lactucopicrin, lactucopicrin, dihydro-lactucin-15-oxalate, lactucin-15-oxalate and dihydro-8-deoxy-lactucin-15-oxalate. A suitable method to determine the sesquiterpene lactone content, defined as the combined amount (i.e. the sum) of lactucin, dihydro-lactucin, 8-deoxylactucin-15-oxalate, 8-deoxylactucin, dihydro-8-deoxylactucin, lactucopicrin-15-oxalate, dihydro-lactucopicrin-oxalate, dihydro-lactucopicrin, lactucopicrin, dihydro-lactucin-15-oxalate, lactucin-15-oxalate and dihydro-8-deoxy-lactucin-15-oxalate is by a chromatographic method, such as ultra-high pressure liquid chromatography coupled to mass spectrometry (UHPLC-MS). A preferred method to determine the sesquiterpene lactone content is in accordance with the UHPLC-MS protocol described herein. Thus, in embodiments, the methods and products of the invention are provided, having the sesquiterpene lactone content characteristics as described herein when determined in accordance with the UHPLC-MS protocol described herein.

As used herein, the term 'sugars' refers to the monosaccharides glucose and fructose as well as the disaccharides fructose-fructose and glucose-fructose. Thus, in view of the definitions provided herein, sucrose is included in the term 'sugars' and excluded from the term 'inulin'. A suitable method to determine the sugar content, defined as the combined amount (i.e. the sum) of glucose monosaccharide, fructose monosaccharides, fructose-fructose disaccharides and glucose-fructose disaccharides is by a chromatographic method such as gel permeation chromatography coupled to a refractive index detector (GPC-RI). A preferred method to determine the sugar content is in accordance with the GPC-RI protocol described herein. Thus, in embodiments, the methods and products of the invention are provided, having the sugar content characteristics as described herein when determined in accordance with the GPC-RI protocol described herein.

As used herein, the term 'nanofiltration' (sometimes referred to in the art as ultrafiltration) refers to filtration using membranes with a pore size of less than 10 nm.

As used herein, the term 'microfiltration' refers to filtration using membranes with a pore size of 0.1-10 μm.

Dry matter content is determined based on the weight loss after drying in a hot air oven for 20 hours at 80°C followed by 2 hours at 105 °C.

Protein content as referred to herein is determined using the Kjehldahl method with a conversion factor of 6.25.

As used herein, the term 'minerals' refers to chloride, bromide, nitrate, malate, sulfate, oxalate, phosphate, potassium, sodium, calcium and magnesium. As will be appreciated by those skilled in the art, these are minerals naturally present in the source of the sesquiterpene lactones and present in the aqueous liquid to be purified in the methods as defined herein. Stated differently, these are minerals naturally present in the plant material that is extracted to provide the aqueous liquid to be purified in the methods as defined herein. A suitable method to determine the mineral content, defined as the combined amount (i.e. the sum) of chloride, bromide, nitrate, malate, sulfate, oxalate, phosphate, potassium, sodium, calcium and magnesium, is by determining the anion content by chromatography, such as high pressure ion chromatography coupled to a conductivity detector (HPIC-CD) and by determining the cation content by inductively coupled plasma - atomic emission spectroscopy (ICP-AES) analysis. A preferred method to determine the mineral content is in accordance with the HPIC-CD protocol described herein (for the anions) and in accordance with the ICP-AES protocol described herein (for the cations). Thus, in embodiments, the methods and products of the invention are provided, having the mineral content characteristics as described herein when determined in accordance with the HPIC-CD protocol described herein (for the anions) and in accordance with the ICP-AES protocol described herein (for the cations).

As used herein the term '*polyphenols*' refers to one or more of 4-O-caffeoylquinate, chlorogenic acid, caffeic acid and cichoric acid. The polyphenols content as used herein concerns the combined amount (i.e. the sum) of 4-O-caffeoylquinate, chlorogenic acid, caffeic acid and cichoric acid. A suitable method to determine the polyphenols content, defined as the combined amount (i.e. the sum) of 4-O-caffeoylquinate, chlorogenic acid, caffeic acid and cichoric acid is by liquid chromatography coupled to mass spectrometry detection (LC-MS). A preferred method to determine the polyphenols content is in accordance with the LC-MS protocol described herein. Thus, in embodiments, the methods and products of the invention are provided, having the polyphenols content characteristics as described herein when determined in accordance with the LC-MS protocol described herein.

As used herein, the term '*ion-exchange treatment*' comprises cation exchange using an ion-exchange resin, anion exchange using an ion-exchange resin as well as ion-exchange chromatography. The ion-exchange treatment referred to herein is preferably not a chromatographic treatment but simply a resin treatment wherein resin is contacted with the liquid to be treated followed by separation from the resin.

**The process of the invention**

In a first aspect the present invention provides a process for recovering sesquiterpene lactones, comprising the steps of:

(a) providing an aqueous liquid comprising sesquiterpene lactones and one or more of sugars, minerals, inulin and polyphenols;

(b) subjecting the aqueous liquid of step (a) to a resin adsorption step using a stationary phase comprising a first non-ionic polymer resin, thereby obtaining a sesquiterpene lactones loaded resin and an adsorption eluate; and

(c) desorbing sesquiterpene lactones from the loaded resin of step (b) and collecting a desorption eluate comprising sesquiterpene lactones.

*The aqueous liquid provided in step (a)*

The aqueous liquid provided in step (a) preferably comprises sesquiterpene lactones and inulin, more preferably the aqueous liquid provided in step (a) comprises sesquiterpene lactones, inulin and polyphenols. Separation of sesquiterpene lactones from such mixtures are, as has been explained herein before, particularly problematic and arise in the context of inulin production where so far the solution has been to use separation methods from which sesquiterpene lactones cannot be recovered. In highly preferred embodiments, the aqueous liquid comprises sesquiterpene lactones, sugars, minerals, inulin and polyphenols. Such aqueous liquids are typically crude chicory root extracts and may further comprise components such as organic acids (described herein in more detail elsewhere) and amino acids. Thus, the sesquiterpene lactones are preferably chicory sesquiterpene lactones. Suitable and preferred chicory plants have been described herein earlier.

Hence, in preferred embodiments of the invention the aqueous liquid provided in step (a) is a plant material extract or originates from a plant material extract, preferably the aqueous liquid provided in step (a) is a plant material extract from a plant of the Asteraceae family or originates from a plant material extract from a plant of the Asteraceae family. The plant material may be obtained from plant parts selected from the group consisting of leaves, roots, flowers, seeds and combinations thereof, more preferably from the leaves or the roots, most preferably from the root.

In highly preferred embodiments the aqueous liquid provided in step (a) is a chicory extract or originates from a chicory extract, preferably the aqueous liquid provided in step (a) is a chicory root extract or originates from a chicory root extract. Suitable and preferred chicory plants have been described herein earlier. The plant roots are preferably provided in the form of pulp.

In preferred embodiments of the invention, the aqueous liquid provided in step (a) comprises more than 1 wt.% (by total weight of aqueous liquid), preferably more than 5 wt.%, more preferably more than 8 wt.% of inulin. In embodiments, the aqueous liquid provided in step (a) comprises 1-30 wt.%, preferably 5-20 wt.%, more preferably 5-15 wt.% of inulin. In embodiments, the inulin comprised in the aqueous liquid provided in step (a) has a number-average degree of polymerization (DP) in the range of 3-60, preferably within the range of 5-30, preferably within the range of 8-20.

The aqueous liquid provided in step (a) preferably has a dry matter content of at least 2% (w/w), preferably at least 5% (w/w), more preferably at least 8% (w/w). The aqueous liquid provided in step (a) preferably has a dry matter content within the range of 2-40% (w/w), preferably within the range of 5-30% (w/w), more preferably within the range of 8-15% (w/w). The inulin content is preferably at least 60 wt.% (based on dry matter), preferably at least 70 wt.% (based on dry matter).

In embodiments, the aqueous liquid provided in step (a) comprises more than 1 wt.% (based on dry matter) minerals, preferably more than 2.5 wt.% of minerals.

In embodiments the aqueous liquid provided in step (a) comprises more than 1 wt.% (based on dry matter), preferably more than 2.5 wt.% of organic acids, wherein the organic acids are preferably chosen from the group consisting of citric acid, malic acid, lactic acid, formic acid, acetic acid, propionic acid, butyric acid and combinations thereof.

In preferred embodiments the aqueous liquid provided in step (a) comprises less than 1 vol%, preferably less than 0.5 vol% of solvents other than water, such as alcohols. In embodiments the aqueous liquid provided in step (a) is substantially free of added solvents other than water, such as alcohols. Minor amounts of solvent such as ethanol may be present due to the natural presence of these molecules in plants, or due to some extent of spontaneous fermentation taking place upon storage of the material, these are not considered added solvents.

In some embodiments, the aqueous liquid provided in step (a) and the inulin comprised therein have not been subjected to a hydrolysis treatment, such as an enzymatic hydrolysis treatment. In other embodiments, the aqueous liquid provided in step (a) and the inulin comprised therein have been subjected to a hydrolysis treatment, such as an enzymatic hydrolysis treatment.

In embodiments, the aqueous liquid provided in step (a) comprises more than 30 mg/kg (by total weight), preferably more than 60 mg/kg (by total weight), more preferably more than 120 mg/kg (by total weight) of sesquiterpene lactones. In embodiments, the aqueous liquid provided in step (a) comprises more than 0.04 wt.% (by weight of inulin), preferably more than 0.1 wt.%, more preferably more than 0.15 wt.% of sesquiterpene lactones. In embodiments, more than 10 wt.% (by weight of the sesquiterpene lactones), preferably more than 20 wt.% of the sesquiterpene lactones comprised in the aqueous liquid provided in step (a) are lactucins.

In embodiments, the aqueous liquid provided in step (a) comprises more than 0.5 wt.% (by weight of inulin), preferably more than 1.5 wt.%, more preferably more than 3 wt.% of sugars. In embodiments, the aqueous liquid provided in step (a) comprises more than 0.5 wt.% (based on dry matter), preferably more than 1.5 wt.%, more preferably more than 3 wt.% of sugars.

In embodiments the aqueous liquid provided in step (a) comprises more than 0.05 wt.% (by weight of inulin), preferably more than 0.1 wt.% of polyphenols. In embodiments the aqueous liquid provided in step (a) comprises sesquiterpene lactones and polyphenols in a ratio (w/w) of 5:1 to 1:5, preferably in a ratio of 3:1 to 1:3.

In embodiments the aqueous liquid provided in step (a) has a pH of between 4 and 8, preferably between 4.5 and 7, more preferably between 5 and 6.5.

Thus, in preferred embodiments of the invention, the aqueous liquid provided in step (a):

- comprises sesquiterpene lactones and inulin; and

- comprises more than 0.04 wt.% (by weight of inulin), preferably more than 0.1 wt.%, more preferably more than 0.15 wt.% of sesquiterpene lactones; and

- preferably has an inulin content of at least 60 wt.% (based on dry matter), preferably at least 70 wt.% (based on dry matter).

In preferred embodiments, the aqueous liquid provided in step (a):

- comprises sesquiterpene lactones and inulin; and

- comprises more than 0.04 wt.% (by weight of inulin), preferably more than 0.1 wt.%, more preferably more than 0.15 wt.% of sesquiterpene lactones; and

- comprises more than 0.05 wt.% (by weight of inulin), preferably more than 0.1 wt.% of polyphenols; and

- preferably has an inulin content of at least 60 wt.% (based on dry matter), preferably at least 70 wt.% (based on dry matter).

In preferred embodiments, the aqueous liquid provided in step (a):

- comprises sesquiterpene lactones and inulin; and

- comprises more than 0.04 wt.% (by weight of inulin), preferably more than 0.1 wt.%, more preferably more than 0.15 wt.% of sesquiterpene lactones; and

- comprises more than 0.05 wt.% (by weight of inulin), preferably more than 0.1 wt.% of polyphenols; and

- comprises more than 0.5 wt.% (by weight of inulin), preferably more than 1.5 wt.%, more preferably more than 3 wt.% of sugars; and

- preferably has an inulin content of at least 60 wt.% (based on dry matter), preferably at least 70 wt.% (based on dry matter).

In preferred embodiments, the aqueous liquid provided in step (a):

- comprises sesquiterpene lactones and inulin; and

- comprises more than 0.04 wt.% (by weight of inulin), preferably more than 0.1 wt.%, more preferably more than 0.15 wt.% of sesquiterpene lactones; and

- comprises more than 0.05 wt.% (by weight of inulin), preferably more than 0.1 wt.% of polyphenols; and

- comprises more than 0.56 wt.% (by weight of inulin), preferably more than 1.57 wt.%, more preferably more than 38 wt.% of sugars; and

- comprises more than 1 wt.% (by dry matter) minerals, preferably more than 2.5 wt.% of minerals; and

- preferably has an inulin content of at least 60 wt.% (based on dry matter), preferably at least 70 wt.% (based on dry matter).

In preferred embodiments, the aqueous liquid provided in step (a):

- comprises sesquiterpene lactones and inulin; and

- comprises more than 0.04 wt.% (by weight of inulin), preferably more than 0.1 wt.%, more preferably more than 0.15 wt.% of sesquiterpene lactones; and

- comprises more than 0.05 wt.% (by weight of inulin), preferably more than 0.1 wt.% of polyphenols; and

- comprises more than 0.56 wt.% (by weight of inulin), preferably more than 1.57 wt.%, more preferably more than 38 wt.% of sugars; and

- comprises more than 1 wt.% (by dry matter) minerals, preferably more than 2.5 wt.% of minerals; and

- comprises more than 1 wt.% (by dry matter), preferably more than 2.5 wt.% of organic acids, wherein the organic acids are preferably chosen from the group consisting of citric acid, malic acid, lactic acid, formic acid, acetic acid, propionic acid, butyric acid and combinations thereof; and

- preferably has an inulin content of at least 60 wt.% (based on dry matter), preferably at least 70 wt.% (based on dry matter).

In accordance with preferred embodiments of the invention, the aqueous liquid provided in step (a) has been subjected to microfiltration.

In accordance with some particular embodiments of the invention, the aqueous liquid provided in step (a) comprises or is the permeate of a nanofiltration treatment performed on an aqueous liquid comprising sesquiterpene lactones and one or more of sugars, minerals, inulin and polyphenols. Such nanofiltration permeates (also referred to as "nanofiltrate") still comprise inulin, but in a smaller amount than a crude inulin extract. An advantage of employing such nanofiltration permeates is that in view of their lower inulin content they can be concentrated (for example via reverse osmosis) without precipitation issues before being subjected to step (b) of the process, thereby increasing process efficiency.

In accordance with highly preferred embodiments of the invention, the aqueous liquid provided in step (a) has not been subjected to ion-exchange treatment and/or active carbon filtration. Preferably, the aqueous liquid provided in step (a) has not been subjected to ion-exchange treatment and active carbon filtration. In some embodiments, the process for recovering sesquiterpene lactones of the invention does not comprise ion-exchange treatment and/or active carbon filtration. In some embodiments the process for recovering sesquiterpene lactones of the invention does not comprise ion-exchange treatment and active carbon filtration. In an embodiment, the process for recovering sesquiterpene lactones of the invention does not comprise an ion-exchange treatment and does comprise an active carbon treatment of the adsorption eluate or a derivative thereof obtained in step (b). In an embodiment, the aqueous liquid provided in step (a) has not been subjected to ion-exchange treatment and/or active carbon filtration and the process for recovering sesquiterpene lactones of the invention does not comprise any ion-exchange treatment and does comprise an active carbon treatment of the adsorption eluate or a derivative thereof obtained in step (b). In a very preferred embodiment, the process as defined herein does not comprise a crystallization step.

In embodiments, the aqueous liquid provided in step (a) is obtainable by a method comprising the following steps:

a1) providing a plant material comprising inulin, proteins, sugars and sesquiterpene lactones, preferably plant material from a plant of the Asteraceae family, most preferably chicory root as described herein before;

a2) subjecting the plant material to a processing step to provide an aqueous liquid comprising inulin, sugars, proteins, sesquiterpene lactones and processed plant material;

a3) separating the processed plant material and the aqueous liquid;

a4) optionally subjecting the aqueous liquid to a protein removal step wherein the proteins are at least partially removed; and

a5) optionally subjecting the aqueous liquid obtained in step a3) or in step a4) to a nanofiltration step;

a6) optionally subjecting the nanofiltrate obtained in step a5) to a concentration step, preferably using reverse osmosis;

wherein the aqueous liquid of step a3) or the aqueous liquid of step a4) or the nanofiltrateof step a5) or the concentrated nanofiltrate of step a6) is provided as the aqueous liquid of step (a). In some preferred embodiment steps a1), a2), a3), a4) and a5) are performed. In some preferred embodiment steps a1), a2), a3), a4), a5) and a6) are performed.

Step a1) typically comprises subjecting the plant material to mechanical operations such as cutting, crushing, shredding, milling, or the like in order to yield a particulate plant material comprising particles such as strips, slices or cubes typically having an average major dimension in the range of 0.1-10 cm, preferably 0.5-5 cm. Preferably slices having a with an average product thickness of 0.5-10 mm, preferably 1-5 mm are used. Step a1) may comprise a coarse physical purification step intended to remove rocks and debris. In embodiments the coarse physical purification step comprises filtration using a screen with aperture larger than 50 $\mu$m, preferably larger than 90 $\mu$m, preferably larger than 100 $\mu$m. Step a1) may also comprise washing of the plant material, typically with (tap) water at ambient temperature.

Step a2) may be performed in many ways in order to provide an aqueous liquid comprising inulin, sugars, proteins and sesquiterpene lactones. In accordance with the invention, step a2) preferably comprises a processing step selected from the group consisting of thermal aqueous extraction, pulsed electric field treatment, increased pressure treatment, reduced pressure treatment, fermentation, acidification, freezing/thawing, size reduction, enzyme-assisted extraction, ultrasound treatment, microwave treatment, supercritical fluid extraction and combinations thereof. In very preferred embodiments, step a2) comprises a processing step selected from the group consisting of thermal aqueous extraction and/or pulsed electric field treatment. In case increased inulin production is desired, thermal aqueous extraction is preferred, while in case increased sesquiterpene lactone production is desired, a cold method such as pulsed electric field treatment is preferred. The thermal aqueous extraction is preferably performed at more than 55 °C, while a cold method such as pulsed electric field is preferably performed at less than 40 °C.

In embodiments, step a2) comprises mashing the plant material, such as chicory roots, in order to make the aqueous liquid comprised in the plant material available for separation/collection.

In highly preferred embodiments, step a2) comprises contacting the plant material with an aqueous extraction liquid for an amount of time sufficient to at least partially extract the inulin. Thus, in preferred embodiments step (a) comprises the following steps:

a1) providing a plant material comprising inulin, proteins, sugars and sesquiterpene lactones, preferably plant material from a plant of the Asteraceae family, most preferably chicory root as described herein before;

a2) contacting the plant material with an aqueous extraction liquid for an amount of time sufficient to provide an aqueous extraction liquid comprising inulin, sugars, proteins, sesquiterpene lactones, and processed plant material;

a3) separating the processed plant material and the aqueous extraction liquid;

a4) optionally subjecting the aqueous liquid to a protein removal step wherein the proteins are at least partially removed; and

a5) optionally subjecting the aqueous liquid obtained in step a3) or in step a4) to a nanofiltration step;

a6) optionally subjecting the nanofiltrate obtained in step a5) to a concentration step, preferably using reverse osmosis;

wherein the aqueous liquid of step a3) or the aqueous liquid of step a4) or the nanofiltrate of step a5) or the concentrated nanofiltrate of step a6) is provided as the aqueous liquid of step (a). In some preferred embodiment steps a1), a2), a3), a4) and a5) are performed. In some preferred embodiment steps a1), a2), a3), a4), a5) and a6) are performed.

In preferred embodiments, the extraction step a2) is performed at a temperature within the range of 55-90 °C, preferably 65-75 °C. In preferred embodiments, the extraction step a2) is a countercurrent extraction step. In embodiments, the aqueous extraction liquid comprises an alcohol, such as ethanol. However, highly preferably the aqueous extraction liquid is water and thus free of solvents other than water. The extraction time is typically between 90 and 240 minutes, such as between 120 and 180 minutes, using 0.8-2 litres of aqueous extraction liquid per kg of plant material.

Step a3) may be performed by any means known to the skilled person, such as pressing, decantation and/or filtration. For example, step a3) may be performed using a screw press or basket press equipped with a screen with suitable pore size, such as 0.1-10 mm, preferably 0.5-5 mm, preferably 1-2 mm.

Step a4), sometimes referred to in the art as a clarification step, may be performed by any means known to the skilled person, such as liming, carbonation, centrifugation, flocculation, filtration (such as microfiltration) and/or filtration with the aid of diatomaceous earth or siliceous earths. In preferred embodiments, step a4) comprises or consists of centrifugation and/or microfiltration. In highly preferred embodiments, step a4) comprises or consists of microfiltration.

In preferred embodiments, step a4) comprises lowering the protein content such that the aqueous liquid of step (a) comprises less than 5 wt.% (by total weight of the inulin) protein, preferably less than 3 wt.%, more preferably less than 1 wt.% protein, wherein the protein content is determined using the Kjehldahl method with a conversion factor of 6.25.

In preferred embodiments, step a5) comprises nanofiltration using a nanofiltration membrane having a molecular weight cut-off value of 0.15-1.5 kDa, preferably 0.15-1.0 kDa and providing the permeate as the aqueous liquid of step (a), optionally after a further concentration step a6). Thus, the nanofiltrate or a concentrate thereof may be subjected to steps (b)-(c) and optional further steps of the process of the invention.

As will be understood from the above, in some embodiments the inulin comprised in the aqueous liquid of step (a) is essentially native inulin (in particular in those embodiments wherein step a(5) or an enzymatic hydrolysis is not performed). Thus, in embodiments, the chain length distribution of the GpyFn inulins comprised in the aqueous liquid of step (a) has one, two, three, four, five or all of the following characteristics:

- 15-40%, preferably 20-35% of the GpyFn has a DP within the range of 3-10;
- 15-30%, preferably 18-28% of the GpyFn has a DP within the range of 11-15;
- 10-25%, preferably 10-20% of the GpyFn has a DP within the range of 16-20;
- 5-20%, preferably 7-15% of the GpyFn has a DP within the range of 21-25;
- 3-15%, preferably 5-10% of the GpyFn has a DP within the range of 26-30;
- 2-12%, preferably 3-8% of the GpyFn has a DP within the range of 31-35;
- 1-10%, preferably 2-6% of the GpyFn has a DP within the range of 36-40;
- 0.5-8%, preferably 0.5-4% of the GpyFn has a DP within the range of 41-45;
- 0.5-10%, preferably 1-6% of the GpyFn has a DP within the range of 46-64;
- 0.1-4%, preferably 0.2-3% of the GpyFn has a DP of more than 64.

In preferred embodiments, the chain length distribution of the GpyFn inulins comprised in the aqueous liquid of step (a) has all of the following characteristics:

- more than 10%, preferably more than 20% of the GpyFn inulins have a DP within the range of 3-10;
- more than 10%, preferably more than 20% of the GpyFn inulins have a DP of more than 15.

In embodiments, the GpyFn inulins comprised in the aqueous liquid provided in step a) have a chain length distribution wherein every DP within the range of 3-60, preferably within the range of 3-40, preferably within the range of 3-30 is present in an amount of at least 0.1%, preferably at least 1%.

*The resin adsorption step (b)*

In accordance with the invention, step (b) comprises contacting the aqueous liquid provided in step (a) with a first non-ionic polymer resin. Such contacting typically and preferably takes place by flowing the aqueous liquid through a packed bed, expanded bed or membrane (preferably a packed bed) comprising or consisting of the non-ionic polymer resin. The aqueous liquid may be circulated multiple times through the resin bed to increase the amount of sesquiterpene lactones that is adsorbed. Alternatively, the aqueous liquid may be mixed with loose resin particles (such as beads), wherein the permeate is obtained after separating the resin particles by a solid-liquid separation operation such as filtration.

The present inventors have found that while the non-ionic polymer resins show some adsorption affinity for polyphenols, the polyphenols will be displaced by sesquiterpene lactones. Thus, step (b) preferably comprises contacting sufficient aqueous liquid provided in step (a) with the resin to achieve such displacement and thus obtain the sesquiterpene lactones-loaded resin. In a preferred embodiment of the invention, step (b) comprises subjecting at least 10 bed volumes, preferably at least 20 bed volumes, more preferably at least 30 bed volumes of the aqueous liquid provided in step (a) to resin adsorption using a stationary phase which is a packed or expanded bed comprising or consisting of the non-ionic polymer resin. In embodiments 10-80 bed volumes, preferably 20-60 bed volumes, more preferably 30-50 bed volumes of the aqueous liquid provided in step (a) are subjected to resin absorption using a stationary phase which is a packed or expanded bed comprising or consisting of the non-ionic polymer resin in step (b). In this way, a large amount of sesquiterpene lactones can be loaded on the resin before the desorption of step (c) is initiated (optionally after a washing step as is described herein elsewhere).

During the adsorption of step (b), sesquiterpene lactones are adsorbed on or by the non-ionic polymer resin, and the eluate obtained during this adsorption stage is hereafter referred to as the "adsorption eluate". The present inventors have found that the adsorption eluate of step (b) is rich in inulin and thus a valuable process stream which may be further used. This is explained in another section of the present description.

The non-ionic polymer resin employed in step (b) may be porous or non-porous, preferably it is a porous resin. The non-ionic polymer resin is preferably selected from non-ionic polymer resins based on aromatic polymers or acrylic polymers. Examples of suitable non-ionic polymer resins are polystyrenes, styrene-copolymers (for example with divinylbenzene), polyacrylates, and phenol formaldehyde polymers. Most preferably, the non-ionic (porous or non-porous) materials are selected from Amberlite@ XAD-1, XAD-2, XAD-4, XAD-5, XAD-16 (which are styrene-divinylbenzene copolymers manufactured by Dupont) and Diaion® HP10, HP20, HP30, HP40, HP50 (which are styrene-divinylbenzene resins manufactured by Mitsubishi Chemical Co., Japan), Amberlite® XAD-7, XAD-7 HP, XAD-8 or XAD-1180 (which are polyacrylates manufactured by Dupont), and Duolite S-30 (which is a phenol formaldehyde polymer manufactured by Chemical Process Co., U.S.A.). A highly preferred resin for sesquiterpene lactone adsorption is Amberlite® XAD-16.

In accordance with preferred embodiments of the invention, the non-ionic polymer resin is in the form of porous beads. In some embodiments the beads are characterized by a mean size in the range of 0.300 and 0.850 mm, a mean pore diameter size of between 50 - 800 Å, preferably 100-200 Å and/or a surface area in the range of 150-900 $m^2/g$, preferably in the range of 700-900 m2/g.

Step (b) of the process preferably takes place at a temperature within the range of 5-90 °C, preferably 20-90°C, more preferably 50-80 °C, such as 60-75 °C. The present inventors have found that the process can be operated at these temperatures such that no cooling is required in case step (a) comprises a thermal aqueous extraction step (which has been described herein earlier). The aqueous liquid of step (a) is preferably contacted with the resin at a rate of 1-10 bed volumes per hour, such as 2-6 bed volumes per hour.

The present inventors have found that the resin treatment of step (b) can achieve high sesquiterpene lactone adsorption efficiencies as well as high selectiveness for sesquiterpene lactones vis a vis polyphenols. Thus, in preferred embodiments, the ratio of $STL_{feed} : STL_{AE}$ is be more than 4, preferably more than 9, more preferably more than 19 and/or wherein the ratio $PP_{feed} : PP_{AE}$ is less than 3, preferably less than 2, preferably less than 1.5, wherein

$STL_{feed}$ is the amount of sesquiterpene lactones in the aqueous liquid provided in step (a), expressed in mg/kg;

$PP_{feed}$ is the amount of polyphenols in the aqueous liquid provided in step (a), expressed in mg/kg;

$STL_{AE}$ is the amount of sesquiterpene lactones in the adsorption eluate of step (b), expressed in mg/kg;

$PP_{AE}$ is the amount of polyphenols in the adsorption eluate of step (b), expressed in mg/kg.

Due to the high selectivity of the resin treatment of step (b) the present inventors have found that a significant shift in the ratio of sesquiterpene lactones to polyphenols occurs when comparing the aqueous liquid of step (a) with the adsorption eluate of step (b). In preferred embodiments of the invention, the ratio $STL_{feed} : PP_{feed}$ is higher than the ratio $STL_{AE} : PP_{AE}$, wherein

$STL_{feed}$ is the amount of sesquiterpene lactones in the aqueous liquid provided in step (a), expressed in mg/kg;

$PP_{feed}$ is the amount of polyphenols in the aqueous liquid provided in step (a), expressed in mg/kg;

$STL_{AE}$ is the amount of sesquiterpene lactones in the adsorption eluate of step (b), expressed in mg/kg;

$PP_{AE}$ is the amount of polyphenols in the adsorption eluate of step (b), expressed in mg/kg.

In preferred embodiments of the invention, $C_{resin-STL}$ is more than 3, preferably more than 5, more preferably more than 10, most preferably more than 25, wherein

$$C_{resin-STL} = \frac{STL_{feed} \times PP_{AE}}{PP_{feed} \times STL_{AE}}$$

Any reference to the composition of the adsorption eluate herein should be interpreted to refer to the composition of at least one fraction of the eluate, but preferably refers to the composition of the total combined adsorption eluate. Typically, an industrial process will employ two resin beds, alternately directing the feed stream to each bed for adsorption while the other bed undergoes desorption. As such, each bed is alternately undergoing an adsorption stage and a desorption stage, each adsorption stage giving rise to an adsorption eluate which may be collected in fractions or combined to form a total combined adsorption eluate; and each desorption stage giving rise to a desorption eluate which may be collected in fractions or combined to form a total combined desorption eluate.

*Washing of the sesquiterpene lactones loaded resin*

The present inventors have found that sesquiterpene lactones are retained on the resin employed in step (b) when washing with an aqueous solvent comprising more than 80 vol% water. Hence, in preferred embodiments of the invention, the process comprises a washing step between steps (b) and (c), wherein the resin is washed with an aqueous solvent comprising more than 80 vol% water, preferably more than 90 vol% water, more preferably more than 98 vol% water, most preferably the aqueous solvent is water. In the context of the present invention, any reference to water as a solvent includes various grades of purity such as process water (which may for example be sourced from surface water, groundwater, municipal water treatment, local plant water treatment, or a combination thereof), demineralized water, etc.
Such a washing step has been found to be advantageous in order to obtain highly pure sesquiterpene lactone fractions, while at the same time limiting inulin losses, since it removes residual inulin and other residual non-adsorbed components like sugars and salts from the resin as well as may remove small amounts of polyphenols which are adsorbed on the resin, without significantly affecting sesquiterpene lactone yield.
Washing is preferably performed using at least 1 bed volumes of the aqueous solvent, such as 1-8 bed volumes, preferably 1-3 bed volumes, such as about 2 bed volumes. Washing is preferably performed at a same, or comparable, rate as step (b), thus preferably at a rate of 1-10 bed volumes per hour, such as 2-6 bed volumes per hour.
The liquid phase obtained from the optional washing step is referred to herein as "washing eluate".
As the skilled person understands, when switching from loading the resin with aqueous liquid of step (a) to washing the resin or to the desorption of step (c), typically about 1-2 bed volumes having an "intermediate" composition are obtained. For the purposes of the present invention, the first bed volume obtained after the feed to the resin is changed from the aqueous liquid of step (a) to the washing or desorption liquid is still considered as comprised in the adsorption eluate, while the first bed volume obtained after the feed to the resin is changed from the washing liquid to the desorption liquid is still considered as comprised in the washing eluate.

*The desorption step (c)*

In step (c) of the process of the present invention, the sesquiterpene lactones are desorbed from the resin, which gives rise to a desorption eluate comprising sesquiterpene lactones. In accordance with the invention, the desorption eluate may be collected in one or more separate fractions. The present inventors have found that a first fraction of the desorption eluate has a very high sesquiterpene lactone concentration while this quickly reduces in subsequent fractions of desorption eluate collected after the first fraction of desorption eluate, while the other components of the eluate show less variation. Hence, it may be desirable to collect such first desorption fraction separate from subsequent desorption fractions in order to allow an extremely high purity sesquiterpene lactone composition to be provided.

Step (c) of the process of the present invention preferably comprises desorbing sesquiterpene lactones with a solvent comprising an organic solvent and optionally water. The organic solvent is preferably selected from alcohols, esters, organic acids and combinations thereof, more preferably selected from methanol, ethanol, ethyl acetate, or combinations thereof. In preferred embodiments step (c) comprises desorbing sesquiterpene lactones with a solvent comprising water and one or more of methanol, ethanol, and ethyl acetate, more preferably step (c) comprises desorbing sesquiterpene lactones with a solvent comprising water and ethanol. The composition of the solvent used to desorb the sesquiterpene lactones may vary during the desorption step. For example, in some embodiments the solvent comprises water and one or more of methanol, ethanol, and ethyl acetate, wherein a gradient in the solvent composition from a first to a second water concentration is employed during the desorption step (c).

The solvent used for desorbing the sesquiterpene lactones in step (c) of the process may comprise a limited amount of further components or additives, such as salts, acids or bases but preferably comprises at least 90 vol%, more preferably at least 95 vol%, most preferably at least 99 vol% of the organic solvent and optionally water; preferably comprises at least 90 vol%, more preferably at least 95 vol%, most preferably at least 99 vol% of water and one or more of methanol, ethanol, and ethyl acetate, more preferably comprises at least 90 vol%, more preferably at least 95 vol%, most preferably at least 99 vol% of water and ethanol. In highly preferred embodiments, the solvent used for desorbing the sesquiterpene lactones in step (c) of the process consists essentially of the organic solvent and optionally water, preferably of methanol, ethanol, ethyl acetate, and combinations thereof, and optionally water; preferably of water and one or more of methanol, ethanol, ethyl acetate, and combinations thereof, more preferably of water and ethanol.

In a preferred embodiment, the solvent used for desorbing the sesquiterpene lactones in step (c) of the process comprises the organic solvent and water in an organic solvent:water ratio within the range of 20:80 to 80:20, more preferably within the range of 60:40 to 40:60, wherein the organic solvent is preferably selected from alcohols, ethyl acetate, and combinations thereof, more preferably selected from methanol, ethanol, ethyl acetate, and combinations thereof, most preferably ethanol. In a highly preferred embodiment, the solvent used for desorbing the sesquiterpene lactones in step (c) of the process consists essentially of the organic solvent and water in an organic solvent:water ratio within the range of 20:80 to 80:20, more preferably within the range of 60:40 to 40:60, wherein the organic solvent is preferably selected from alcohols, ethyl acetate, and combinations thereof, more preferably selected from methanol, ethanol, ethyl acetate, and combinations thereof, most preferably ethanol.

In a preferred embodiment, the solvent used for desorbing the sesquiterpene lactones in step (c) of the process comprises ethanol and water, in a ratio of ethanol:water within the range of 20:80 to 80:20, more preferably within the range of 60:40 to 40:60. In a most preferred embodiment, the solvent used for desorbing the sesquiterpene lactones in step (c) of the process consists essentially of ethanol and water, in a ratio of ethanol:water within the range of 20:80 to 80:20, more preferably within the range of 60:40 to 40:60.

In an embodiment, the solvent used for desorbing the sesquiterpene lactones in step (c) of the process is substantially free of water.

The solvent used for desorbing the sesquiterpene lactones may be recycled, i.e. recovered from the desorption eluate and re-used in the desorption step.

Step (c) of the process preferably takes place at a temperature within the range of 5-90 °C, preferably 20-75°C, more preferably 30-65 °C, such as 40-60 °C. The desorption solvent in step (c) is preferably contacted with the resin at a rate of 1-10 bed volumes per hour, such as 2-6 bed volumes per hour. Desorption is preferably performed using at least 2 bed volumes of the desorption solvent, such as 2-10 bed volumes, preferably 2-7 bed volumes, most preferably 3-6 bed volumes.

The present inventors have found that the process of the invention does not only allow the removal and recovery of sesquiterpene lactones from the aqueous liquid provided in step (a), it also allows for the recovery of a more concentrated sesquiterpene lactone stream than the starting stream. Thus, in preferred embodiments according to the invention, $STL_{DE} > STL_{feed}$, preferably $STL_{DE} > 2*STL_{feed}$, more preferably $STL_{DE} > 4*STL_{feed}$, wherein

$STL_{DE}$ is the amount of sesquiterpene lactones in the desorption eluate, expressed in mg/kg; and

$STL_{feed}$ is the amount of sesquiterpene lactones in the aqueous liquid provided in step (a), expressed in mg/kg.

The present inventors have found that the process of the invention does not only allow the removal and recovery of sesquiterpene lactones from the aqueous liquid provided in step (a), it also allows for the recovery of a sesquiterpene lactone stream which has a very low inulin content. Thus, in preferred embodiments according to the invention, $IN_{feed} > IN_{DE}$, preferably $IN_{feed} > 5*IN_{DE}$, more preferably $IN_{feed} > 10*IN_{DE}$, wherein

$IN_{feed}$ is the amount of inulin in the aqueous liquid provided in step (a), expressed in wt.%; and

$IN_{DE}$ is the amount of inulin in the desorption eluate, expressed in wt.%. This embodiment is particularly preferred when a washing step as described herein is performed.

**The** present inventors have found that the present process allows the selective isolation from sesquiterpene lactones from polyphenols. Thus, in preferred embodiments according to the invention, $PP_{feed}$:$PP_{DE}$ is more than 3, preferably more than 5, more than 10, and/or $PP_{AE}$:$PP_{DE}$ is more than 3, preferably more than 5, more than 10, wherein

$PP_{feed}$ is the amount of polyphenols in the aqueous liquid provided in step (a), expressed in mg/kg;

$PP_{DE}$ is the amount of polyphenols in the desorption eluate, expressed in mg/kg; and

$PP_{AE}$ is the amount of polyphenols in the adsorption eluate of step (b), expressed in mg/kg. This embodiment is particularly preferred when a washing step as described herein is performed.

**Any** reference to the composition of the desorption eluate herein should be interpreted to refer to the composition of at least one fraction of the desorption eluate, but preferably refers to the composition of the total combined desorption eluate.

*Optional further processing of the desorption eluate obtained in step (c)*

The desorption eluate obtained in step (c) may be used as such, or may be further processed, typically to concentrate it for storage or transportation.

In preferred embodiments, the process of the invention further comprises a step (d) which comprises concentration the desorption eluate obtained in step (c).

Preferably, step (d) comprises concentrating the desorption eluate to provide a sesquiterpene lactone concentrate with a dry matter content of at least 5 wt. %, preferably at least 10 wt.%, more preferably at least 25 wt%.

Concentrating the desorption eluate may be performed by any process or combination of processes known in the art, such as evaporation, precipitation, spray-drying, freeze-drying, drum drying or membrane methods (e.g. reverse osmosis or nanofiltration employing a very low MWCO membrane). In highly preferred embodiments, step d) comprises or consists of evaporation and/or spray-drying, preferably evaporation followed by spray-drying. The temperature during the concentration step preferably does not exceed 80 °C, preferably does not exceed 60 °C, most preferably does not exceed 40 °C.

Step (d) may comprise a step of recovering water and/or organic solvent comprised in the desorption eluate, wherein at least part of the recovered water and/or organic solvent is provided to form at least part of the solvent used for desorbing the sesquiterpene lactones in step (c). In this way, at least partial recycling of solvent and thus greater process efficiency is achieved. Solvent recovery can for example be performed by any of the methods mentioned for concentrating, but is preferably performed by evaporation or distillation, preferably employing a pressure of less than 1 atm such that mild temperatures can be used. Similarly, if solvent removal is performed for the purposes of recycling, the temperature during the concentration step preferably does not exceed 80 °C, preferably does not exceed 60 °C, most preferably does not exceed 40 °C.

In all embodiments of the invention wherein the desorption eluate obtained in step (c) comprises an organic solvent (typically because an organic solvent was contained in the desorption solvent), it is preferred that step (d) comprises removal of the organic solvent, such that a sesquiterpene-lactone containing composition is obtained which is substantially free of organic solvent.

The present inventors have found that some inulin (particularly high DP inulin) may be contained in the desorption eluate obtained in step (c). Thus, step (d) may comprise a step of recovering inulin from the desorption eluate obtained in step (c), for example by nanofiltration, precipitation or crystallization.

Other purification steps may be performed, such as liquid-liquid extraction (e.g. using water, an organic solvent, or mixtures thereof).

In embodiments, step (d) comprises concentrating the desorption eluate to provide a sesquiterpene lactone concentrate which is in the form of an aqueous composition which has a dry matter content of at least 1% (w/w), preferably at least 2.5% (w/w), more preferably at least 10% (w/w), most preferably at least 25% (w/w).

In embodiments, the sesquiterpene lactone concentrate of step d) is in the form of a powder, preferably a spray-dried or freeze-dried powder, which has a dry matter content of more than 80% (w/v), preferably more than 85% (w/v), more preferably more than 90% (w/v), most preferably more than 95% (w/v).

*The adsorption eluate obtained in step (b) and further treatment thereof*

The present inventors have found that the adsorption eluate of step (b) is rich in inulin and thus a valuable process stream which may be further used. It can be considered as an inulin raffinate. The adsorption eluate of step (b) may be used as such, or may be further treated in order to further concentrate and /or purify the inulin contained therein.

In preferred embodiments, the process of the present invention further comprises a step (e) subjecting the adsorption eluate of step (b) to one or more of the following treatments:

- (e1) nanofiltration;

- (e2) precipitation or crystallization;

- (e3) ion-exchange resin treatment and/or active carbon treatment; and

- (e4) electrodialysis.

In some embodiments, the process of the present invention further comprises a step (e) subjecting the adsorption eluate of step (b) to (e1) nanofiltration. Nanofiltration of the adsorption eluate of step (b) gives rise to a retentate which is rich in inulin, and a permeate. The nanofiltration typically serves to remove impurities (such as organic acids, amino acids, minerals, polyphenols, etc.) from the inulin composition. Suitable nanofiltration membranes are those having a molecular weight cut-off value within the range of 0.15-1.5 kDa, preferably 0.15-1.0 kDa. Preferred nanofiltration membranes are those comprising or consisting of one or more polymers selected from the group consisting of polyamide (PA), polysulfone (PS), polyethersulfone (PES), polyimide, and polypiperazine, preferably polyamide (PA) or polypiperazine. By selecting a nanofiltration membrane having a molecular weight cut-off value of less than 2 kDa, removal of sugars can be achieved while low DP inulin, minerals and organic acids can be retained in the retentate, which may be advantageous with a view to nutritional properties. Nanofiltration of inulin is described in more detail in WO2020/182714A1. Preferred membranes are those having a molecular weight cut-off value of less than 1.5 kDa, more preferably less than 1.2 kDa, more preferably less than 1.05 kDa and preferably a molecular weight cut-off value of more than 0.1 kDa, preferably more than 0.15 kDa. The retentate is collected and provided as an inulin product, optionally after further concentration in step (f) as described herein below.

In some embodiments, the process of the present invention further comprises a step (e) subjecting the adsorption eluate of step (b) to (e2) precipitation or crystallization. In accordance with the invention, the precipitation or crystallization refers to precipitation or crystallization of inulin, such that inulin is recovered as solid material after separating the precipitated or crystallized inulin from the precipitation or crystallization liquor by a solid-liquid separation, such as filtration or cyclone separation.

In some embodiments, the process of the present invention further comprises a step (e) subjecting the adsorption eluate of step (b) to (e3) ion-exchange resin treatment and/or active carbon treatment. The ion-exchange treatment may comprise treatment using an anion-exchange resin, a cation-exchange resin or a combination thereof. The present inventors have found that the adsorption eluate stream (b) obtained in the process of the present invention can be efficiently further purified by ion-exchange resin treatment and/or active carbon treatment with longer times until the resin or active carbon is saturated or fouled, leading to increased regeneration intervals and thus higher process efficiency. In some highly preferred embodiments, the adsorption eluate of step (b) is subjected to (e1) nanofiltration and the retentate from nanofiltration step (e1) is subsequently subjected to (e3) ion-exchange resin treatment and/or active carbon treatment.

In embodiments, the method of the present invention further comprises a step (f) of concentrating the adsorption eluate as obtained from step (b) or after the one or more treatments of step (e). Preferably, the method of the present invention further comprises a step (f) of concentrating

- the adsorption eluate obtained from step(b); or

- the retentate of nanofiltration step (e1); or

- the adsorption eluate of step (b) after having been submitted to ion-exchange resin treatment and/or active carbon treatment step (e3); or

- the retentate of nanofiltration step (e1) after having been submitted to ion-exchange resin treatment and/or active carbon treatment step (e3).

Step (f) preferably comprises concentration to provide an inulin concentrate with an inulin concentration of at least 20 wt.% (by total weight of the concentrate), preferably at least 60 wt.%. In preferred embodiments, step (f) comprises concentrating the retentate to provide an inulin concentrate with a dry matter content of at least 50% (w/w), preferably at least 70 wt.% (w/w).

The concentrating of step (f) may be performed by any process or combination of processes known in the art, such as membrane methods (e.g. reverse osmosis or nanofiltration employing a very low MWCO membrane), evaporation, precipitation, spray-drying, freeze-drying or drum drying. In highly preferred embodiments, step d) comprises or consists of evaporation and/or spray-drying, preferably evaporation followed by spray-drying.

In embodiments, the inulin concentrate obtained in step (f) is in the form of an aqueous solution comprising 20-80 wt.%, preferably 30-60 wt.% inulin and which has a dry matter content of less than 85% (w/w), preferably less than 65% (w/w).

In embodiments, the inulin concentrate obtained in step (d) is in the form of a powder, preferably a spray-dried or freeze-dried powder, comprising more than 80 wt.%, preferably more than 85 wt.%, more preferably more than 90 wt.%, most preferably more than 95 wt.% inulin and which has a dry matter content of more than 80% (w/w), preferably more than 85% (w/w), more preferably more than 90% (w/w), most preferably more than 95% (w/w).

In embodiments, the process does not comprise an enzymatic hydrolysis treatment, such that the inulin comprised in the concentrate of step (f) has not been subjected to enzymatic hydrolysis. In embodiments, the process does comprise an enzymatic hydrolysis treatment, such that the inulin comprised in the concentrate of step (f) has been subjected to enzymatic hydrolysis.

*Polyphenol recovery*

**The** present inventors have found that the selective sesquiterpene lactone recovery from the aqueous liquid provided in step (a) allows a selective polyphenol recovery to be performed.

In some embodiments of the invention, the process comprises a step

(g) subjecting a polyphenol-containing stream to a resin adsorption step using a stationary phase comprising a non-ionic polymer resin, thereby obtaining a polyphenol loaded resin and an eluate,

wherein the polyphenol-containing stream is selected from

- the aqueous liquid of step (a); and/or

- the washing liquid of the optional washing step described herein;

- and/or the adsorption eluate of step (b) or a derivative thereof.

Preferably, the polyphenol-containing stream is the aqueous liquid of step (a) and/or the adsorption eluate of step (b), preferably the polyphenol-containing stream is the adsorption eluate of step (b) or a derivative thereof. Examples of derivatives of the adsorption eluate of step (b) are the streams obtained by subjecting the adsorption eluate of step (b) to optional further processing steps (e) and/or (f). If the polyphenol-containing stream is the adsorption eluate of step (b) or a derivative thereof, then it is preferred that step (g) takes place before the optional steps (e) and (f). In other words, a step of polyphenol removal from the inulin-rich stream preferably takes place before the optional further purification and concentration of steps (e) and (f), in particular because the purification of step (e3) would also remove polyphenols without the option of recovering the polyphenols.

In preferred embodiments of the invention, the ratio $PP_{g1}:STL_{g1}$ is higher than the ratio $PP_{g2}$ $STL_{g2}$, wherein

$STL_{g1}$ is the amount of sesquiterpene lactones in the polyphenol-containing stream of step (g), expressed in mg/kg;

$PP_{g1}$ is the polyphenol-containing stream of step (g), expressed in mg/kg;

$STL_{g2}$ is the amount of sesquiterpene lactones in the eluate of step (g), expressed in mg/kg; and

$PP_{g2}$ is the amount of polyphenols in the eluate of step (g), expressed in mg/kg.

In preferred embodiments of the invention, $C_{resin-PP}$ is more than 3, preferably more than 5, more preferably more than 10, most preferably more than 25, wherein

$$C_{resin-PP} = \frac{STL_{g2} \times PP_{g1}}{PP_{g2} \times STL_{g1}}$$

Any reference to the composition of the eluate of step (g) herein should be interpreted to refer to the composition of at least one fraction of the eluate of step (g), but preferably refers to the composition of the total combined eluate of step (g).

The present inventors have found that non-ionic polymer resins as have been described herein before in the context of step (b) exhibit polyphenol adsorption but have a greater affinity for sesquiterpene lactones, such that after running sufficient bed volumes through the resin, the sesquiterpene lactones will displace the polyphenols and a highly selective sesquiterpene lactone separation can be achieved. However, this also means that the same resins can be used to recover polyphenols from the polyphenol-rich stream, in particular if it is low in sesquiterpene lactones, such as when it is the polyphenol-containing stream is the aqueous liquid of step (a) and/or the eluate of step (b), preferably the polyphenol-containing stream is the eluate of step (b). The resin is thus preferably a non-ionic polymer resin as described herein in the context of step (b). Thus, step (g) comprises subjecting a polyphenol-containing stream to a resin adsorption step using a stationary phase comprising a second non-ionic polymer resin, thereby obtaining a polyphenol loaded resin and an eluate, wherein the second non-ionic polymer resin may be the same or different from the first non-ionic polymer resin.

Step (g) preferably comprises desorbing sesquiterpene lactones from the polyphenol loaded resin and collecting a desorption eluate comprising polyphenols. Desorption preferably takes place using an aqueous solvent comprising more than 80 vol% water, preferably more than 90 vol% water, more preferably more than 98 vol% water, most preferably the aqueous solvent is water.

Said desorption eluate comprising polyphenols obtained from step (g) may be concentrated in a similar manner as has been described herein before for the sesquiterpene lactone-rich desorption eluate obtained in step (c).

**Sesquiterpene lactone composition of the invention**

In another aspect, the invention provides a sesquiterpene lactone-containing composition obtainable by the process described herein. Preferably the sesquiterpene lactone-containing composition is obtainable as the desorption eluate of step (c) of the process described herein or a concentrate thereof, for example as is obtainable from step (d) of the process as has been described herein before.

Such sesquiterpene lactone-containing composition, when compared to e.g. an organic solvent chicory root extract, is characterized amongst others by a high sesquiterpene lactone content in combination with a low sugar content and/or a low polyphenols content. The sesquiterpene lactones are preferably chicory sesquiterpene lactones. Suitable and preferred chicory plants have been described herein earlier.

Thus, in another aspect the invention provides a sesquiterpene lactone composition comprising sesquiterpene lactones and at least one of polyphenols and sugars,

- wherein the ratio (w/w) of sesquiterpene lactones to sugars is at least 2:1, preferably at least 5:1, more preferably at least 10:1; and/or

- wherein the ratio (w/w) of sesquiterpene lactones to polyphenols is at least 10:1, preferably at least 25:1, more preferably at least 100:1, most preferably at least 1000:1.

The sesquiterpene lactone composition may further comprise inulin, wherein the ratio (w/w) of sesquiterpene lactones to inulin is preferably at least 0.05:1, preferably at least 0.1:1, more preferably at least 0.15:1.

In some embodiments, for example when the aqueous liquid provided in step (a) comprises or consists of a nanofiltrate, as described herein earlier, very low inulin concentrations can be achieved. Thus, in some embodiments the sesquiterpene lactone composition further comprises inulin, wherein the ratio (w/w) of sesquiterpene lactones to inulin is at least 0.5:1, preferably at least 1:1, more preferably at least 5:1.

The sesquiterpene lactone composition preferably comprises at least 3 wt.% (based on dry matter) of sesquiterpene lactones, preferably at least 5 wt.%, more preferably at least 7.5 wt.%.

The dry matter content of the sesquiterpene lactone composition is preferably at least 5 wt.%, preferably at least 10 wt.%.

In embodiments, the sesquiterpene lactone composition is in the form of an aqueous composition which has a dry matter content of at least 1% (w/w), preferably at least 2.5% (w/w), more preferably at least 10% (w/w), most preferably at least 25% (w/w).

In embodiments, the sesquiterpene lactone composition is in the form of a powder, preferably a spray-dried or freeze-dried powder, which has a dry matter content of more than 80 wt.%, preferably more than 85wt.%, more preferably more than 90wt.%, most preferably more than 95wt.%.

Thus, in preferred embodiments of the invention there is provided a sesquiterpene lactone composition comprising sesquiterpene lactones and at least one of polyphenols and sugars,

- wherein the ratio (w/w) of sesquiterpene lactones to sugars is at least 2:1, preferably at least 5:1, more preferably at least 10:1; and

- wherein the ratio (w/w) of sesquiterpene lactones to polyphenols is at least 500:1, preferably at least 1000:1, more preferably at least 1500:1; and

- comprising inulin, wherein the ratio (w/w) of sesquiterpene lactones to inulin is preferably at least 0.05:1, preferably at least 0.1:1, more preferably at least 0.15:1; and

- comprising at least 3 wt.% (by dry matter) of sesquiterpene lactones, preferably at least 5 wt.%, more preferably at least 7.5 wt.%; and

- wherein preferably the dry matter content of the sesquiterpene lactone composition is at least 5% (w/v), preferably at least 10 wt.% (w/v).

In embodiments, the sesquiterpene lactone composition comprises one, two, three, four or all of the following:

- 2-35 wt.% (by dry matter) of sesquiterpene lactones, preferably 4-25 wt.%;

- 10-50 wt.% (by dry matter) of inulin, preferably 20-40 wt.%;

- less than 6 wt.% (by dry matter) of sugars, preferably less than 2 wt.%;

- less than 0.1 wt.% (by dry matter) of polyphenols, preferably less than 0.01 wt.%;

- less than 6 wt.% (by dry matter) of minerals, preferably less than 3 wt.%.

In embodiments, the sesquiterpene lactone composition comprises

- 2-35 wt.% (by dry matter) of sesquiterpene lactones, preferably 4-25 wt.%; and

- 10-50 wt.% (by dry matter) of inulin, preferably 20-40 wt.%; and

- less than 6 wt.% (by dry matter) of sugars, preferably less than 2 wt.%; and

- less than 0.1 wt.% (by dry matter) of polyphenols, preferably less than 0.01 wt.%; and

- less than 6 wt.% (by dry matter) of minerals, preferably less than 3 wt.%; and

- optionally 10-50 wt.% (by dry matter) of protein, preferably 15-40 wt.%.

In embodiments of the invention there is provided a sesquiterpene lactone composition comprising sesquiterpene lactones and at least one of polyphenols and sugars,

- wherein the ratio (w/w) of sesquiterpene lactones to sugars is at least 2:1, preferably at least 5:1, more preferably at least 10:1; and

- wherein the ratio (w/w) of sesquiterpene lactones to polyphenols is at least 500:1, preferably at least 1000:1, more preferably at least 1500:1; and

- comprising inulin, wherein the ratio (w/w) of sesquiterpene lactones to inulin is preferably at least 0.05:1, preferably at least 0.1:1, more preferably at least 0.15:1; and

- comprising at least 3 wt.% (by dry matter) of sesquiterpene lactones, preferably at least 5 wt.%, more preferably at least 7.5 wt.%; and

- wherein preferably the dry matter content of the sesquiterpene lactone composition is at least 5% (w/v), preferably at least 10 wt.% (w/v); and

- comprising 2-35 wt.% (by dry matter) of sesquiterpene lactones, preferably 4-25 wt.%; and

- comprising 10-50 wt.% (by dry matter) of inulin, preferably 20-40 wt.%; and

- comprising less than 6 wt.% (by dry matter) of sugars, preferably less than 2 wt.%; and

- comprising less than 0.1 wt.% (by dry matter) of polyphenols, preferably less than 0.01 wt.%; and

- comprising less than 6 wt.% (by dry matter) of minerals, preferably less than 3 wt.%; and

- comprising optionally 10-50 wt.% (by dry matter) of protein, preferably 15-40 wt.%.

The sesquiterpene lactone composition may further comprise other components, typically plant material, such as fats, oils, pectins and/or organic acids.

**Inulin-rich composition of the invention**

In another aspect, the invention provides an inulin-rich composition obtainable by the process described herein. Preferably the inulin-rich composition is obtainable as the adsorption eluate of step (b) of the process described herein or a derivative thereof, for example after having been subjected to optional steps (e) and/or (f) as has been described herein before.

Such inulin-rich composition is characterized amongst others by a very low sesquiterpene lactone content compared to the inulin content. The sesquiterpene lactones are preferably chicory sesquiterpene lactones. Suitable and preferred chicory plants have been described herein earlier.

Thus, in another aspect the invention provides an inulin-rich composition comprising inulin, preferably chicory inulin, wherein the composition comprises less than 1000 mg sesquiterpene lactones per kg of inulin, preferably less than 500 mg sesquiterpene lactones per kg of inulin, more preferably less than 200 mg sesquiterpene lactones per kg of inulin. In some highly preferred embodiments the invention provides an inulin-rich composition comprising inulin, preferably chicory inulin, wherein the composition comprises less than 150 mg sesquiterpene lactones per kg of inulin, preferably less than 120 mg sesquiterpene lactones per kg of inulin, more preferably less than 100 mg sesquiterpene lactones per kg of inulin.

The inulin-rich composition preferably comprises at least 50 wt.% (by dry matter) inulin, preferably at least 65 wt.%, more preferably at least 70 wt.%.

The inulin-rich composition may be a liquid composition having a dry matter content of less than 40 % (w/w), such as within the range of 1-30 % (w/w), preferably within the range of 5-20 % (w/w). Such compositions are also known in the art as "debittered inulin juice" and can be stored until further processing or used as is in e.g. the food industry. Alternatively, the inulin-rich composition may be a concentrate having a dry matter content of more than 40 % (w/w), such as a liquid concentrate having a dry matter content of 40-85% (w/w), preferably 40-65% (w/w); or such as a powder, preferably a spray-dried or freeze-dried powder, having a dry matter content of more than 80% (w/w), preferably more than 85% (w/w), more preferably more than 90% (w/w), most preferably more than 95% (w/w).

In some embodiments, the inulin-rich composition further comprises sugars. In such embodiments, the ratio of inulin:sugars in the inulin-rich composition is preferably within the range of 5:1 to 30:1, preferably within the range of 8:1 to 25:1, more preferably within the range of 9:1 to 18:1.

In some embodiments, the inulin-rich composition further comprises minerals. In such embodiments, the amount of minerals in the inulin-rich composition is preferably at least 1 wt.% (based on dry matter), preferably at least 2 wt.% (based on dry matter). In the embodiments wherein step (a) comprises nanofiltration, as described herein earlier, relative amount of minerals will be significantly higher (in view of the removal of inulin by nanofiltration). Thus, in some embodiments the amount of minerals in the inulin-rich composition is preferably at least 5 wt.% (based on dry matter), preferably at least 10 wt.% (based on dry matter). Such high mineral but low sesquiterpene lactone compositions may offer unique nutritional value while still having a good taste.

The inulin-rich composition may further comprise other components, typically plant material, such as fats, oils, organic acids, pectins and amino acids.

**EXAMPLES**

The concentrations of the different components provided in the experimental section and anywhere else herein, have been determined in accordance with the below analysis protocols.

*HPIC-CD protocol:* The concentration of chloride, bromide, nitrate, malate, sulfate, oxalate and phosphate were determined using high pressure ion chromatography coupled with a conductivity detector (HPIC-CD). A calibration curve was constructed by analysing a series of dilutions of a stock solution containing chloride, bromide, nitrate, phosphate, malate, sulfate and oxalate (100 mg/kg each in demineralized water). Measurements were performed using the following setup (employing a KOH gradient in the eluent).

| Autosampler | Thermo AS-AP |
|---|---|
| Injection volume | 7 μL |
| Injector temperature | 15 °C |
| Pump flow | 0.38 ml/min |
| Eluent generator | EGC 500 KOH |
| Column | Dionex IonPac AS11-HC-4μm |
| Max. pressure | 5000 psi |
| Column temperature | 35 °C |
| Supressor | AERS-500 2mm |
| Supressor current | 52 mA |
| Detector | Thermo Fisher Scientific Integrion Analytical CD |
| Detector temperature | 30 °C |
| Analysis time | 37.5 min |

| Step | Time (min) | Concentration (mM KOH) |
|---|---|---|
| 1 | 0 | 10 |
| 2 | 8 | 10 |
| 3 | 21 | 26.25 |
| 4 | 33 | 50 |
| 5 | 33 | 75 |
| 6 | 35 | 75 |
| 7 | 35 | 10 |
| 8 | 37.5 | 10 |

*ICP-AES protocol:* sodium, potassium, calcium and magnesium concentrations were determined using inductively coupled plasma - atomic emission spectroscopy (IPC-AES) analysis. Analyte samples were acidified using HNOs and measured in a type Thermo Fisher Scientific iCap 6000 series ICP emission spectrometer using 1150W RF power. The following solutions (acidified using HNOs) were used for calibration purposes.

| Stock solution | Ca (mg/kg) | K (mg/kg) | Mg (mg/kg) | Na (mg/kg) |
|---|---|---|---|---|
| blank | 0 | 0 | 0 | 0 |
| 1 | 1.25 | 25 | 1.25 | 25 |
| 2 | 2.50 | 50 | 2.50 | 50 |
| 3 | 3.75 | 100 | 3.75 | 100 |

(continued)

| Stock solution | Ca (mg/kg) | K (mg/kg) | Mg (mg/kg) | Na (mg/kg) |
|---|---|---|---|---|
| 4 | 5 | 150 | 5 | 150 |

*GPC-RI protocol:* monosaccharides, disaccharides, DP3 inulin, DP4 inulin, DP5 inulin and >DP5 inulin were determined using gel permeation chromatography coupled to a refractive index detector (GPC-RI). A calibration solution was prepared containing 0.5%(w/v) fructose, 0.5%(w/v) glucose, 0.5%(w/v) saccharose. Another calibration solution containing an in-house inulin/ polyfructofuranose reference standard was prepared. Calibration curves were constructed by analysing a series of dilutions of the calibration solutions. Sample peaks in the chromatogram were integrated manually after visual inspection in order to prevent acids and salts to be interpreted as inulin. Measurements were performed using the following setup.

| | |
|---|---|
| Injection volume | 20 μl |
| Column | Shodex KS-802, 300 x 8 mm |
| Guard column | Shodex KS-800P 50 x 6 mm |
| Column temperature | 50 °C |
| Max. pressure | 50 bar |
| Eluent | MilliQ water |
| Flow | 1.0 ml/min |
| Detector | Shodex RI-101, max range |
| Detector temperature | 35 °C |

*UHPLC-MS protocol:* Characterization and quantification of dihydro-lactucin-15-oxalate, lactucin-15-oxalate, dihydro-lactucin, lactucin, 8-deoxy-lactucin-15-oxalate, dihydro-8-deoxy-lactucin-15-oxalate, 8-deoxy-lactucin, dihydro-8-deoxy-lactucin, dihydro-lactucopicrin-15-oxalate, lactucopicrin-15-oxalate, dihydro-lactucopicrin, and lactucopicrin were determined using Ultra High Pressure Liquid Chromatography coupled with triple quadrupole Mass Spectrometry (UHPLC-MS). A calibration curve was constructed by analysing a dilution series of a calibration solution containing an in-house sesquiterpene lactones standard. 1 ml sample was diluted with 3 ml MeOH (5% acetic acid), and 1 ml sesquiterpene lactones standard was added. Measurements were performed using an Agilent® 1260 UHPLC coupled to an Agilent®6420 ESI-QQQ-MS (electron spray ionization - triple quad - mass spectrometer) using the following setup. Quantitative calculations were made using Agilent Masshunter software.

| UHPLC | |
|---|---|
| Pump | flow 0.4 ml/min |
| Injection volume | 5 ml |
| Eluent | Acetonitrile (0.1% acetic acid) gradient:<br>1- 8 min: 20 à 50% ACN<br>8- 9 min: 50% ACN<br>8- 9 min: 50 à 20% ACN<br>Runtime: 12min |
| Column oven temperature | 40°C |
| **MS** | |
| Mode | MRM |
| Polarity | Positive + Negative |
| Delta EMV (+) | 200 |
| Delta EMV (-) | 200 |
| Gas temperature | 300°C |

(continued)

| MS | |
|---|---|
| Gas flow | 11 l/min |
| Nebulizer | 45 psi |
| Capillary | 3000 V |

*LC-MS protocol:* 4-O-caffeoylquinate, chlorogenic acid, caffeic acid and cichoric acid were determined using liquid chromatography coupled to UV (330 nm) and subsequent mass spectrometry detection (LC-MS). Samples were diluted using 0.1% formic acid in water. Measurements were performed using an Agilent 1260 with the following setup.

| Column | Poroshell 120 SB-C18 (2.1 x 150 mm, 2.7 $\mu$m, Agilent |
|---|---|
| Column temperature | 25°C |
| Eluent A | 0.1% formic acid in water |
| Eluent B | Acetonitrile |
| Injection volume | 5$\mu$l |

Gradient

| Time (min) | %A | %6B | Flow (mL/min) |
|---|---|---|---|
| 0 | 95 | 5 | 0.30 |
| 1 | 92 | 8 | 0.30 |
| 2 | 89 | 11 | 0.30 |
| 3 | 85 | 15 | 0.30 |
| 5 | 84 | 16 | 0.30 |
| 9 | 83 | 17 | 0.30 |
| 10 | 82 | 18 | 0.30 |
| 14 | 79 | 21 | 0.30 |
| 15 | 60 | 40 | 0.30 |
| 16 | 0 | 100 | 0.30 |
| 17 | 95 | 5 | 0.30 |
| 20 | 95 | 5 | 0.30 |

*HPAEC-PAD protocol:* the chain length distribution of inulin was determined using high performance anion exclusion chromatography coupled to pulsed amperometric detection (HPAEC-PAD). Inulin samples were stabilized using 1 ml NaOH (1M) and diluted to 1% (w/v) inulin. After heating in a hot water bath until all inulin is dissolved, the hot sample is filtered over a 0.45$\mu$m membrane filter and diluted 10x for analysis. Hydrolyzed inulin samples were diluted to 1% (w/v) and diluted 50x for analysis. Inulin response factors according to Timmermans et al. (Timmermans, J. W., van Leeuwen, M. B., Tournois, H., de Wit, D. and Vliegenthart, J. F. G. (1994) 'Quantitative Analysis of the Molecular Weight Distribution of Inulin by Means of Anion Exchange HPLC with Pulsed Amperometric Detection', Journal of Carbohydrate Chemistry, 13:6, 881 - 888) were used. Measurements were performed using a Thermo Fisher Scientific, ICS 5000 ED using the following setup. Chain length distribution was expressed in % of inulin/GpyFn/FpyFn of a specific chain length, wherein the % is calculated based on peak surface area for that chain length compared to total peak surface area.

| Injection volume | 10 $\mu$l |
|---|---|

(continued)

| Column | CarboPac PA200 Analytical (3 $\times$ 250 mm) |
|---|---|
| Max. pressure | 4000 psi |
| Flow | 0.5 ml/min |
| Temperature | 20°C |
| Detector | 30°C, Carbohydrate waveform, Ag/AgCl reference |

Gradiënt inulin sample)

| Tijd | A [ %] (demineralized water) | B [ %] (0,10 M NaOH) | C [ %] (1 M NaAc + 0,10 M NaOH) | Flow ml/min |
|---|---|---|---|---|
| -5 | 0 | 100 | 0 | 0.5 |
| 0 | 0 | 100 | 0 | 0,5 |
| 60 | 0 | 50 | 50 | 0,5 |
| 61 | 0 | 100 | 0 | 0,5 |
| 0 | 0 | 100 | 0 | 0,5 |

Gradiënt (hydrolyzed inulin sample)

| Tijd | A [ %] (demineralized water) | B [ %] (0,10 M NaOH) | C [ %] (1 M NaAc + 0,10 M NaOH) | Flow ml/min |
|---|---|---|---|---|
| 0 | 0 | 100 | 0 | 0.5 |
| 35 | 0 | 70 | 30 | 0,5 |
| 36 | 0 | 100 | 0 | 0,5 |

**Example 1**

Cichorium intybus L. var. sativum DC roots were washed to remove soil, rocks etc. and sliced to thin strips/cossettes using a drum slicer. The chicory root strips were subject to countercurrent extraction using water at 70 °C. The extract was subsequently submitted to clarification and protein removal by microfiltration, to obtain a permeate which is an aqueous solution comprising inulin, sesquiterpene lactones, sugars and minerals which was used as the feed stream for a non-ionic polymer resin and is referred to in the examples as "the chicory extract".

The chicory extract was fed to a packed bed of Amberlite® XAD-16 resin obtained from Dupont, with a bed volume (BV) of 200 ml. 8 L of the chicory extract was loaded onto the resin by feeding at 4 BV/hr over a time of 10 hours at a temperature of ca. 70 °C. During this loading, the sesquiterpene lactones are adsorbed on the resin. Thereafter, the column is washed to remove non-absorbed inulin. This was done using water as wash solvent at a feed of 4 BV/hr, for 30 minutes at a temperature of ca. 50 °C. Once non-absorbed components have been washed off, the desorption of the sesquiterpene lactones takes place. This was achieved by a desorption solvent comprising 50 % v/v ethanol and 50 % v/v water, fed through the column at 4 BV/hr for a time of 75 minutes at a temperature of ca. 50 °C. Eluate was collected in fractions of one bed volume, and each fraction was analyzed for composition. The composition of the various fractions is shown in Table 1.

The desorption eluate fractions BVD1-5 were combined and concentrated in a rotavap at 60°C to obtain the sesquiterpene lactone concentrate BVD1-5 and analyzed.

The adsorption eluate fractions are collected and combined to form the inulin-rich composition BV1-40 and analyzed.

TABLE 1

| | Feed | Adsorption eluate | | | | | Washing | Desorption eluate | | | | | Washing | Concentrate |
|---|---|---|---|---|---|---|---|---|---|---|---|---|---|---|
| | Feed | BV8 | BV16 | BV24 | BV32 | BV40 | Washing 1 | BVD 1 | BVD2 | BVD3 | BVD4 | BVD5 | Washing 2 | BVD1-5 |
| Dry matter (%) | 13.6 | 13.8 | 14.3 | 14.6 | 14.7 | 14.2 | 3.2 | 1.1 | 3.3 | 0.5 | 0.1 | 0.1 | 0 | 12.9 |
| Sesquiterpene lactones (mg/kg) | 337 | 1 | 6 | 12 | 12 | 16 | 22 | 92 | 5540 | 1958 | 140 | 30 | 1 | 11573 |
| Inulin (%) | 11 | 11.2 | 12.2 | 12.1 | 12.1 | 11.2 | 2.4 | 0.3 | 0.4 | 0.1 | 0.1 | 0.1 | 0.1 | 3.5 |
| Sugars (%) | 0.8 | 0.8 | 0.8 | 0.8 | 0.8 | 0.8 | 0.1 | <0.1 | <0.1 | <0.1 | <0.1 | <0.1 | <0.1 | 0.1 |
| Protein (%) | 0.59 | | | | | | | | | | | | | 3,3 |
| Cations (mg/kg) (K, Na, Ca, Mq) | 2870 | | | | | 2860 | | | | | | | | 1440 |
| Anions (mg/kg) (Cl, Br, NO3, SO4, PO4, oxalate) | | | | | | 1928 | | | | | | | | |
| Polyphenols (mg/kg) | 222 | 274 | 273 | 273 | 262 | 220 | 211 | 8 | 2 | 9 | 8 | 1 | 1 | 6 |

**Comparative example 2**

The composition of a chicory root having a typical dry matter content of 24.8% w/w as taken from literature is shown in table 2.

TABLE 2

| Component | Content (% w/w based on dry matter) |
|---|---|
| Sesquiterpene lactones | 0.5 |
| Sugars | 24.6 |
| Salts | 2.5 |
| Inulin | 61.1 |
| Polyphenols | 0.7 |

**Comparative Example 3**

Whole chicory root tubers were washed and sliced to obtain chicory root slices having an average size of a few centimeters. The chicory slices were subsequently extracted using ethyl acetate at ca. 50 °C and the extract was concentrated by evaporation to a dry matter content of about 90% (w/w). The composition of the extract obtained is shown in Table 3.

TABLE 3

| Component | Content (% w/w based on dry matter) |
|---|---|
| Sesquiterpene lactones | 19.7 |
| Sugars | 14.3 |
| Salts | 1.3 |
| Inulin | 34.1 |
| Polyphenols | 0.1 |

**Example 4**

The experiment of example 1 was repeated using Amberlite® XAD-7 HP instead of Amberlite® XAD16 as well as 2L (10 BV) of feed and adsorption and desorption rate of 2 BV/hr. The resulting composition is shown in the below table.

TABLE 4

| | Feed | Adsorption eluate | Washing | Desorption eluate | | | | Washing | Concentrate |
|---|---|---|---|---|---|---|---|---|---|
| | | BV1-10 | Washing 1 | BVD1 | BVD2 | BVD3 | BVD1-5 | Washing 2 | BVD1-5 |
| **Dry matter (%)** | 13.2 | 12.8 | 0.0 | 0.2 | 0.3 | 0.0 | 0.4 | 0.0 | 2.6 |
| **Sesquiterpene lactones (mg/kg)** | 160 | 5.0 | | 201 | 1005 | 28.8 | 474 | <1 | 2889 |
| **Inulin (%)** | 12.0 | 11.7 | <0.1 | <0.1 | | | | | |
| **Sugars (%)** | 0.9 | | | | | | | | |
| **Cations (mg/kg)** (K, Na, Ca, Mg) | | | | | | | | | 254 |

(continued)

|  | Feed | Adsorption eluate | Washing | Desorption eluate |  |  |  | Washing | Concentrate |
|---|---|---|---|---|---|---|---|---|---|
|  |  | BV1-10 | Washing 1 | BVD1 | BVD2 | BVD3 | BVD1-5 | Washing 2 | BVD1-5 |
| Anions (mg/kg) (Cl, Br, NO3, SO4, PO4, oxalate) |  |  |  |  |  |  |  |  | 150 |
| Polyphenols (mg/kg) | 259 | 225 |  | 10 | 8.4 | <1 | 11.4 | <1 | 130 |

## Example 5

The experiment of example 1 was repeated using a chicory extract further purified by nanofiltration to separate minerals, sesquiterpene lactones and other small molecules from the inulin. The inulin-lean nanofiltrate was taken and was concentrated using reverse osmosis before being used as feed. 4.2L (21 BV, 2BV/hr) of feed was processed.

|  | Feed | Adsorption eluate |  | Washing | Concentrate |
|---|---|---|---|---|---|
|  | Feed | BV6 | BV21 | Washing 1 | BVD1-5 |
| Dry matter (%) | 6.0 | 6.3 | 6.2 | 0.3 | 9.7 |
| Sesquiterpene lactones (mg/kg) | 396 | 1.5 | 10.1 | 19.0 | 12835 |
| Inulin (%) | 1.2 |  |  |  | <0.1 |
| Sugars (%) | 1.2 |  |  |  | <0.1 |
| Protein (%) | 1.0 |  |  |  | 0.6 |
| Cations (mg/kg) (K, Na, Ca, Mq) | 6310 |  |  |  | 316 |
| Polyphenols (mg/kg) | 556 | 16 | 434 | 212 | 556 |

## Claims

1.   A process for recovering sesquiterpene lactones, comprising the steps of:

(a) providing an aqueous liquid comprising sesquiterpene lactones and one or more of sugars, minerals, inulin and polyphenols;
(b) subjecting the aqueous liquid of step (a) to a resin adsorption step using a stationary phase comprising a first non-ionic polymer resin, thereby obtaining a sesquiterpene lactones loaded resin and an adsorption eluate; and
(c) desorbing sesquiterpene lactones from the loaded resin of step (b) and collecting a desorption eluate comprising sesquiterpene lactones.

2.   Process according to claim 1, wherein the aqueous liquid provided in step (a) is a plant material extract or originates from a plant material extract, preferably the aqueous liquid provided in step (a) is a plant material extract from a plant of the Asteraceae family or originates from a plant material extract from a plant of the Asteraceae family, more preferably the aqueous liquid provided in step (a) is chicory extract or originates from a chicory extract.

3.   Process according to claim 2, wherein step (a) comprises the steps of

a1) providing a plant material comprising inulin, proteins, sugars and sesquiterpene lactones, preferably chicory root;
a2) subjecting the plant material to a processing step to provide an aqueous liquid comprising inulin, sugars, proteins, sesquiterpene lactones and processed plant material;

a3) separating the processed plant material and the aqueous liquid; and

a4) optionally subjecting the aqueous liquid to a protein removal step wherein the proteins are at least partially removed to provide the aqueous liquid of step (a); and

a5) optionally subjecting the aqueous liquid obtained in step a3) or in step a4) to a nanofiltration step;

a6) optionally subjecting the nanofiltrate obtained in step a5) to a concentration step, preferably using reverse osmosis;

wherein the aqueous liquid of step a3) or the aqueous liquid of step a4) or the nanofiltrate of step a5) or the concentrated nanofiltrate of step a6) is provided as the aqueous liquid of step (a).

4. Process according to any one of the previous claims wherein the aqueous liquid of step (a) comprises inulin and polyphenols.

5. Process according to any one of the previous claims, preferably according to claim 4, comprising a washing step between steps (b) and (c), wherein the resin is washed with an aqueous solvent comprising more than 80 vol% water, preferably water.

6. Process according to any one of the previous claims wherein the ratio $STL_{feed}$:$STL_{AE}$ is more than 4, preferably more than 9, more preferably more than 19 and/or wherein the ratio $PP_{feed}$:$PP_{AE}$ is less than 3, preferably less than 2, preferably less than 1.5, wherein

$STL_{feed}$ is the amount of sesquiterpene lactones in the aqueous liquid provided in step (a), expressed in mg/kg;
$PP_{feed}$ is the amount of polyphenols in the aqueous liquid provided in step (a), expressed in mg/kg;
$STL_{AE}$ is the amount of sesquiterpene lactones in the adsorption eluate of step (b), expressed in mg/kg; and
$PP_{AE}$ is the amount of polyphenols in the adsorption eluate of step (b), expressed in mg/kg.

7. Process according to any one of the previous claims wherein the ratio $STL_{feed}$:$PP_{feed}$ is higher than the ratio $STL_{AE}$:$PP_{AErm}$, wherein

$STL_{feed}$ is the amount of sesquiterpene lactones in the aqueous liquid provided in step (a), expressed in mg/kg;
$PP_{feed}$ is the amount of polyphenols in the aqueous liquid provided in step (a), expressed in mg/kg;
$STL_{AE}$ is the amount of sesquiterpene lactones in the adsorption eluate of step (b), expressed in mg/kg; and
$PP_{AE}$ is the amount of polyphenols in the adsorption eluate of step (b), expressed in mg/kg.

8. Process according to claim 7 wherein $C_{resin-STL}$ is more than 3, preferably more than 5, more preferably more than 10, most preferably more than 25, wherein

$$C_{resin-STL} = \frac{STL_{feed} \times PP_{perm}}{PP_{feed} \times STL_{perm}}.$$

9. Process according to any one of the previous claims, wherein the step (c) comprises desorbing sesquiterpene lactones with a solvent comprising an organic solvent and optionally water, wherein the organic solvent is preferably selected from methanol, ethanol, ethyl acetate, and combinations thereof, preferably wherein the organic solvent is ethanol.

10. Process according to any one of the previous claims further comprising a step
(e) subjecting the adsorption eluate of step (b) to one or more of the following treatments:

- (e1) nanofiltration;
- (e2) precipitation or crystallization;
- (e3) ion-exchange resin treatment and/or active carbon treatment; and
- (e4) electrodialysis.

11. A sesquiterpene lactone composition comprising sesquiterpene lactones and at least one of polyphenols and sugars,

• wherein the ratio (w/w) of sesquiterpene lactones to sugars is at least 2:1, preferably at least 5:1, more preferably at least 10:1; and/or

- wherein the ratio (w/w) of sesquiterpene lactones to polyphenols is at least 500:1, preferably at least 1000:1, more preferably at least 1500:1.

12. The composition of claim 11 further comprising inulin, wherein the ratio (w/w) of sesquiterpene lactones to inulin is at least 0.05:1, preferably at least 0.1:1, more preferably at least 0.15:1.

13. The composition of claim 11 or 12 comprising at least 3 wt.% (by dry matter) of sesquiterpene lactones, preferably at least 5 wt.%, more preferably at least 7.5 wt.%; and wherein preferably the dry matter content of the sesquiterpene lactone composition is at least 5 wt.% , preferably at least 10 wt.%.

14. A composition comprising inulin, preferably chicory inulin, wherein the composition comprises less than 1000 mg sesquiterpene lactones per kg of inulin, preferably less than 500 mg sesquiterpene lactones per kg of inulin, more preferably less than 200 mg sesquiterpene lactones per kg of inulin.

15. The composition of claim 14 comprising less than 120 mg sesquiterpene lactones per kg of inulin, more preferably less than 100 mg sesquiterpene lactones per kg of inulin and at least 1 wt.% (based on dry matter) of minerals.

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FEDERICO FERIOLI ET AL: "An update procedure for an effective and simultaneous extraction of sesquiterpene lactones and phenolics from chicory", FOOD CHEMISTRY, ELSEVIER LTD, NL, vol. 135, no. 1, 14 April 2012 (2012-04-14), pages 243-250, XP028491734, ISSN: 0308-8146, DOI: 10.1016/J.FOODCHEM.2012.04.079 [retrieved on 2012-04-21] * 2.3, 2.4, * | 1-10 | INV. C07D307/93 A01N65/12 A61K31/343 |
| X | WO 2020/049173 A1 (SENSUS B V [NL]) 12 March 2020 (2020-03-12) * claim 1; table 4 * | 1-10 | |
| X | CN 103 864 736 A (LANZHOU CHEM PHYS INST) 18 June 2014 (2014-06-18) * the whole document * | 1-10 | |
| X,D | WO 2020/182714 A1 (SENSUS B V [NL]) 17 September 2020 (2020-09-17) * claim 1 * | 1-10 | **TECHNICAL FIELDS SEARCHED (IPC)** C07D A01N A61K |
| X | EP 1 179 299 A2 (NESTLE SA [CH]) 13 February 2002 (2002-02-13) * tables 1,2 * | 1-10 | |
| X | WO 2012/094636 A2 (ELCELYX THERAPEUTICS INC [US]; BARON ALAIN D [US] ET AL.) 12 July 2012 (2012-07-12) * paragraphs [0012], [0107], [0330] * | 1-10 | |

-/--

The present search report has been drawn up for all claims

| Place of search | Date of completion of the search | Examiner |
|---|---|---|
| Munich | 16 April 2024 | Baston, Eckhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

Europäisches
Patentamt

European
Patent Office

Office européen
des brevets

## EUROPEAN SEARCH REPORT

**Application Number**

EP 23 20 8210

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAN GE: "Version updates of strategies for drug discovery based on effective constituents of traditional Chinese medicine", ACUPUNCTURE AND HERBAL MEDICINE, vol. 3, no. 3, 3 July 2023 (2023-07-03), pages 158-179, XP093151970, ISSN: 2097-0226, DOI: 10.1097/HM9.0000000000000071 * figures 1-3 * | 1-10 | |
| X | CN 103 316 087 A (DONG YU) 25 September 2013 (2013-09-25) * the whole document * | 1-10 | |

**TECHNICAL FIELDS SEARCHED (IPC)**

The present search report has been drawn up for all claims

| Place of search | Date of completion of the search | Examiner |
|---|---|---|
| Munich | 16 April 2024 | Baston, Eckhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................................
& : member of the same patent family, corresponding document

EPO FORM 1503 03.82 (P04C01)

page 2 of 2

Europäisches
Patentamt

European
Patent Office

Office européen
des brevets

**Application Number**

EP 23 20 8210

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-10

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-10

   Process for isolating sesquiterpene lactones
   - - -

2. claims: 11-13

   Sesquiterpene lactone composition comprising at least one of polyphenols and sugars of defined ratio
   - - -

3. claims: 14, 15

   Inulin composition having less than 1000 mg sesquiterpene lactone
   - - -

## ANNEX TO THE EUROPEAN SEARCH REPORT
## ON EUROPEAN PATENT APPLICATION NO.

EP 23 20 8210

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2024

| Patent document cited in search report | | Publication date | Patent family member(s) | | Publication date |
|---|---|---|---|---|---|
| WO 2020049173 | A1 | 12-03-2020 | DK | 3846627 T3 | 06-03-2023 |
| | | | EP | 3846627 A1 | 14-07-2021 |
| | | | ES | 2940332 T3 | 05-05-2023 |
| | | | LT | 3846627 T | 11-04-2023 |
| | | | PL | 3846627 T3 | 17-04-2023 |
| | | | WO | 2020049173 A1 | 12-03-2020 |
| CN 103864736 | A | 18-06-2014 | NONE | | |
| WO 2020182714 | A1 | 17-09-2020 | CL | 2021002313 A1 | 23-09-2022 |
| | | | EP | 3935088 A1 | 12-01-2022 |
| | | | US | 2022177607 A1 | 09-06-2022 |
| | | | WO | 2020182714 A1 | 17-09-2020 |
| EP 1179299 | A2 | 13-02-2002 | AU | 8588201 A | 25-02-2002 |
| | | | EP | 1179299 A2 | 13-02-2002 |
| | | | WO | 0213625 A2 | 21-02-2002 |
| WO 2012094636 | A2 | 12-07-2012 | AU | 2012204162 A1 | 01-08-2013 |
| | | | AU | 2017206190 A1 | 03-08-2017 |
| | | | AU | 2019253780 A1 | 07-11-2019 |
| | | | AU | 2022201075 A1 | 10-03-2022 |
| | | | BR | 112013017411 A2 | 20-09-2016 |
| | | | CA | 2823397 A1 | 12-07-2012 |
| | | | CL | 2013001982 A1 | 10-01-2014 |
| | | | CN | 103597071 A | 19-02-2014 |
| | | | CY | 1123590 T1 | 24-03-2022 |
| | | | DK | 2661266 T3 | 16-11-2020 |
| | | | EA | 201370154 A1 | 30-01-2014 |
| | | | EP | 2661266 A2 | 13-11-2013 |
| | | | EP | 3763419 A1 | 13-01-2021 |
| | | | ES | 2834986 T3 | 21-06-2021 |
| | | | HR | P20201731 T1 | 25-12-2020 |
| | | | HU | E051738 T2 | 29-03-2021 |
| | | | JP | 2014501787 A | 23-01-2014 |
| | | | KR | 20140035331 A | 21-03-2014 |
| | | | LT | 2661266 T | 10-12-2020 |
| | | | PT | 2661266 T | 30-11-2020 |
| | | | SG | 191855 A1 | 30-08-2013 |
| | | | SG | 10201607085W A | 28-10-2016 |
| | | | SI | 2661266 T1 | 29-01-2021 |
| | | | US | 2012177730 A1 | 12-07-2012 |
| | | | US | 2014030332 A1 | 30-01-2014 |
| | | | US | 2015065578 A1 | 05-03-2015 |
| | | | US | 2015265555 A1 | 24-09-2015 |
| | | | US | 2017020829 A1 | 26-01-2017 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 23 20 8210

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2024

| Patent document cited in search report | Publication date | Patent family member(s) | | Publication date |
|---|---|---|---|---|
| | | US 2019038576 A1 | | 07-02-2019 |
| | | WO 2012094636 A2 | | 12-07-2012 |
| | | ZA 201304702 B | | 25-11-2015 |
| | | ZA 201606957 B | | 30-05-2018 |
| CN 103316087 A | 25-09-2013 | NONE | | |

For more details about this annex : see Official Journal of the European Patent Office, No. 12/82

page 2 of 2

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020182714 A1

**Non-patent literature cited in the description**

- **TIMMERMANS, J. W. ; VAN LEEUWEN, M. B. ; TOURNOIS, H. ; DE WIT, D. ; VLIEGENTHART, J. F. G.** Quantitative Analysis of the Molecular Weight Distribution of Inulin by Means of Anion Exchange HPLC with Pulsed Amperometric Detection. *Journal of Carbohydrate Chemistry*, 1994, vol. 13 (6), 881-888